# EUROPEAN PATENT APPLICATION

(11) **EP 2 332 569 A2**
(43) Date of publication of application: **15.06.2011**
(21) Application number: 10010617.8
(22) Date of filing: 27.06.2005
(51) Int. Cl.: A61K 39/00, A61K 39/02, A61K 39/095, A61K 39/39, A61P 37/02

(54) **Compositions and methods for treating neurological disorders**

(30) Priority: 25.06.2004 US 582999 P
(62) Divisional of application: 05787543.7
(71) Applicant: ID Biomedical Corporation of Quebec, Laval, QC H7V 3S8 (CA); The Brigham and Women's Hospital, Inc., Boston MA 02115 (US)
(72) Inventor: Frenkel, Dan, Dr., Rehovot 76283 (IL); Maron, Ruth, Rehovot 76100 (IL); Burt, David, Dollard des Omeaux Québec H9A 3K1 (CA); Weiner, Howard, L., Brookline MA 02445 (US)
(74) Representative: Grünecker, Kinkeldey, Stockmair & Schwanhäusser Anwaltssozietät

(57) **Abstract**

Compositions useful for treating neurological disorders including neurodegenerative disorders associated with deleterious protein aggregation, aberrant protein folding, such as brain amylogenic diseases, and/or neurodegenerative autoimmune disorders are described. Methods of using said compositions also are described. In particular, methods to treat a neurodegenerative disorder such as Alzheimer's disease and a neurodegenerative autoimmune disorder such as Multiple Sclerosis are contemplated utilizing proteosomes and/or Glatiramer Acetate, wherein the GA is in a submicron emulsion or a nanoemulsion.

## Description

This invention was made with government support under a grant awarded by the Department of Health and Human Services. The government has certain rights in this invention.

### CROSS-REFERENCES TO RELATED APPLICATIONS

This application claims benefit of priority from U.S. Provisional Patent Application 60/582,999, filed June 25, 2004, which is hereby incorporated in its entirety as if fully set forth.

### FIELD OF THE INVENTION

Compositions useful for treating neurological disorders including neurodegenerative disorders associated with deleterious protein aggregation, aberrant protein folding and/or neurodegenerative autoimmune disorders such as brain amylogenic diseases are described. Methods of using said compositions also are described.

### BACKGROUND OF THE INVENTION

Neurological diseases are generally characterized by the loss of neurons from one or more regions of the central nervous system. Examples of neurological diseases include Alzheimer's disease, neurofibromatosis, Huntington's disease, depression, amyotrophic lateral sclerosis, Multiple Sclerosis, stroke, Parkinson's disease, and multi-infarct dementia. They are complex in both origin and progression, and have proved to be some of the most difficult types of disease to treat. In fact, for some neurological diseases, there are no drugs available that provide significant therapeutic benefit. The difficulty in providing therapy is all the more tragic given the devastating effects these diseases have on their victims.

Alzheimer's disease (AD) is a degenerative brain disorder characterized clinically by progressive loss of memory, cognition, reasoning, judgment and emotional stability that gradually leads to profound mental deterioration and ultimately death. AD is a very common cause of progressive mental failure (dementia) in aged humans and is believed to represent the fourth most common medical cause of death in the United States. AD has been observed in all races and ethnic groups worldwide and presents a major present and future public health problem. The disease is currently estimated to affect about four million individuals in the United States alone. AD is at present incurable. The administration of certain therapies has been used to treat symptoms of AD in humans. However, no treatment that effectively prevents AD or reverses its symptoms or course in humans is currently known.

The brains of individuals with AD exhibit characteristic lesions, termed senile plaques, and neurofibrillary tangles. Senile plaques characteristic of AD are most frequently localized extracellularly while neurofibrillary tangles are most frequently localized intracellularly. Large numbers of these lesions are generally found in patients with AD in several areas of the human brain important for memory and cognitive function. Smaller numbers of these lesions in a more restricted anatomical distribution are sometimes found in the brains of aged humans who do not have clinical AD. The principal chemical constituent of the senile plaques and vascular amyloid deposits (amyloid angiopathy) characteristic of AD is a protein designated amyloid-β peptide (Aβ, which may also be referred to as βAP, AβP or β/A4. Extracellular plaques containing Aβ may be dense or diffuse. Dense plaques are often referred to as fibrillar plaques. Aβ was first purified and a partial amino acid sequence reported in Glenner and Wong (1984) Biochem. Biophys. Res. Commun. 120:885-890. The isolation procedure and the sequence data for the first 28 amino acids are described in U.S. Pat. No. 4,666,829. Forms of Aβ having amino acids beyond number 40 were first reported by Kang et al. (1987) Nature 325:733-736.

Neuropathologically, AD is characterized, to varying degrees, by four major lesions:
a) intraneuronal, cytoplasmic deposits of neurofibrillary tangles (NFT), b) parenchymal amyloid deposits called neuritic plaques, c) cerebrovascular Aβ amyloidosis (*e.g*., amyloid angiopathy), and d) synaptic and neuronal loss. One of the key events in AD is the deposition of amyloid (*e.g*., Aβ peptide) as insoluble fibrous masses (amyloidogenesis) resulting in extracellular neuritic plaques and deposits around the walls of cerebral blood vessels. The major constituent of the neuritic plaques and cerebral amyloid angiopathy is Aβ, although these deposits also may contain other proteins such as glycosaminoglycans and apolipoproteins.

Solomon, B. et al. (1997) PNAS 94(8):4109-1.2 showed that monoclonal antibody against the N-termini of Aβ can bind to and disaggregate preexisting assemblies of Aβ-peptide and/or prevent fibril aggregation *in vitro* and prevent toxicity to neuronal cell cultures. Schenk, D. et al., Nature 400(6740):173-177 (1999) demonstrated that immunization with amyloid-β attenuated Alzheimer's disease-like pathology in PDAPP transgenic mice serving as an animal model for amyloid-β deposition and Alzheimer's disease-like neuropathologies. They reported that immunization of young animals prior to the onset of Alzheimer's disease-type neuropathologies essentially prevented the development of β-amyloid plaque formation, neuritic dystrophy and astrogliosis, whereas treatment in older animals after the onset of Alzheimer's disease-type neuropathologies was observed to reduce the extent and progression of these neuropathologies. This effect is mediated by antibodies, since peripherally administered antibodies against Aβ have been shown to reduce brain parenchymal amyloid burden (Bard F. et al., (2000) Nat. Med. 6(8):916-9). In addition, intranasal immunization with freshly solubilized Aβ1-40 reduces cerebral amyloid burden (Weiner, H.L. et al., (2000) Ann. Neuro. 48(4):567-79). Two studies, performed by Morgan, D. et al., (2000) Nature 408(6815):982-5; and Janus, C. et al., (2000) Nature 408(6815):979-82, using animal model systems demonstrated that a vaccination-induced reduction in brain amyloid deposits resulted in cognitive improvements. Additional studies have addressed various aspects of the same topic, including Dodart et al., (2002) Nat. Neuroscience 5(5):452-7, and Kotilinek, L.A. et al., (2002) J. Neuroscience 22(15):6331-5. Although Aβ vaccination has shown some success in various studies using animal models of AD, human clinical studies immunizing with Aβ 1-40/42 peptides formulated in an adjuvant (QS21) were terminated because of deleterious and/or an unacceptably high occurrence of side effects such as meningoencephalitis. Thus, there is a need for therapeutically acceptable modalities for the treatment and/or prevention of AD and related neurodegenerative disorders associated with protein aggregation.

Autoimmune diseases are characterized by an abnormal immune response directed to self or autologous tissues. Based on the type of immune response (or immune reaction) involved, autoimmune diseases in mammals can generally be classified into one of two different types: cell-mediated (i.e., T-cell-mediated) or antibody-mediated disorders. Multiple Sclerosis (MS) is a T-cell mediated autoimmune disease (Trapp et al. New Eng. J. Med. 338(5):278 (1998)). More than 1,000,000 young adults worldwide between the ages of thirty and forty have MS. MS is the most common disease of the central nervous system and is the most common cause of neurological disability in young adults. Pathophysiologically, circulating autoreactive T cells mediate much of the central nervous system destruction seen in MS patients (Rudick et al. New Eng. J. Med. 337:1604(1997)).

In MS, T-cells react with myelin basic protein (MBP) which is a component of myelin in the central nervous system. The demonstration that activated T-cells specific for MBP can be isolated from MS patients supports the proposition that MS is an autoimmune disease wherein T-cells destroy the self or autologous neural tissue (Allegretta et al. Science: 247: 778 (1990)).

MS is currently treated with certain anti-inflammatory and immunosuppressive agents, such agents include: (i) corticosteroids, which have both immunomodulatory and immunosuppressive effects; (ii) interferon-β; (iii) glatiramer acetate (GA); (iv) azathioprine, a purine analog which depresses both cell-mediated and humoral immunity; (v) intravenous immune globulin; (vi) methotrexate, which inhibits dihydrofolate reductase and depresses cell-mediated and humoral immunity; (vii) cyclophosphamide, an alkylating agent which has cytotoxic and immunosuppressive effects; and (viii) cyclosporine, which has potent immunosuppressive effects by inhibiting T cell activation. Despite treatment with such anti-inflammatory or immunosuppressive drugs, more than 50% of the patients with MS steadily deteriorate as a result of focal destruction of the spinal cord, cerebellum, and cerebral cortex.

Many of the drugs currently used to treat MS have limited long-term efficacy, in part, because they have significant cytotoxic effects. For example, prolonged treatment with cyclophosphamide can lead to alopecia, nausea, vomiting, hemorrhagic cystitis, leukopenia, myocarditis, infertility, and pulmonary interstitial fibrosis. Treatment with immunosuppressive agents can eventually induce "global" immunosuppression in the treated patient, which greatly increase the risk of infection. Patients subjected to prolonged global immunosuppression have an increased risk of developing severe medical complications from treatment, such as malignancies, kidney failure and diabetes.

An alternative approach to the treatment of MS is the use of intravenous or oral administration of MBP to modulate the T-cell immune response that may be associated therewith. Intravenous administration of MBP or fragments thereof containing immunodominant epitopes of MBP suppresses the immune system by causing clonal anergy, or T-cell unresponsiveness, which deactivates T-cells specific for MBP. The end-result is that MBP-specific T cells no longer proliferate in response to MBP. The inability of the T-cell to proliferate results in a decrease in T-cell mediated destruction of neural tissues.

An immunochemical analog of MBP used in treating MS is glatiramer acetate (GA), or copolymer-1 (COP-1) (U.S. Pat. No. 3,849,550; PCT Application WO/95/31990). GA, in its commercially available form, is a mixture of random synthetic polypeptides composed of L-alanine, L-glutamic acid, L-lysine and L-tyrosine in a molar ratio of 6.0:1.9:4.7:1.0. It was first synthesized as an immunochemical mimic of MBP. For example, certain monoclonal antibodies to GA cross-react with MBP (Teitelbaum et al. Proc. Natl. Acad. Sci. USA 88:9258 (1991)). Also, GA has been found to induce T suppressor cells specific for MBP (Lando et al. J. Immunol. 123:2156 (1979)). Experiments in mice indicate that GA also specifically inhibits MBP-specific T cells that are involved in the destruction of central nervous system tissue in Experimental Allergic Encephalomyelitis (EAE) (Teitelbaum et al. Proc. Natl. Acad. USA 85:9724 (1995)).

Administration of GA may: (i) increase the percentage of NK cells; (ii) reduce serum IL-2 receptors; (iii) suppress TNF-α; and (iv) increase TGF-β and IL-4 (Ariel et al. Multiple Sclerosis 3(5), S053 (1997)).

Although patients with MS have been relatively successfully treated with parenterally administered GA (Bornstein et al. Transactions American Neurological Association, 348 (1987)), the current treatment regime and overall effects could be improved.

Citation of the above documents is not intended as an admission that any of the foregoing is pertinent prior art. All statements as to the date or representation as to the contents of these documents is based on the information available to the applicant and does not constitute any admission as to the correctness of the dates or contents of these documents.

### SUMMARY OF THE INVENTION

The present invention provides methods and compositions for treating neurological diseases or disorders in mammals in need of such treatment. Said neurological diseases or disorders can be associated with a systemic or localized deposition of protein or proteinaceous material (*e.g*., amyloidosis), deleterious protein aggregation (protein mis-folding) and/or neurodegenerative autoimmunity. Particular interest is in the amyloid forming diseases such as Alzheimer's disease and/or other brain amylogenic diseases including prion-related diseases, Huntington disease, Parkinson's disease and cerebral amyloid angiopathy (CAA) (Revesz, T. et al. (2003) J. Neuropathol. Exp. Neurol. 62(9):885-98). The treatment of said amyloid related diseases can include preventing new amyloid plaque (deposition) formation, maintaining current amyloid plaque levels, and/or decreasing the amount of existing amyloid plaque or total brain amyloid protein (including Aβ that may not be deposited into plaques) as measured by determining total amyloid load (soluble and non-soluble Aβ) or the amount of fibrillar Aβ-amyloid load. Said neurological diseases or disorders can be associated with a cell-mediated autoimmune disease such as Multiple Sclerosis. The treatment of said autoimmune disorders can include preventing the formation of autoreactive T cells, maintaining current autoreactive T cell concentrations, and/or decreasing the concentration of autoreactive T cells.

The present invention claims and utilizes various formulations of a proteosome based composition, and/or a GA composition, optionally in a submicron emulsion, or a nanoemulsion, as therapeutics for treating neurological diseases or disorders in mammals including Aβ plaque related diseases or disorders, and cell-mediated autoimmune diseases or disorders.

### BRIEF DESCRIPTION OF THE DRAWINGS

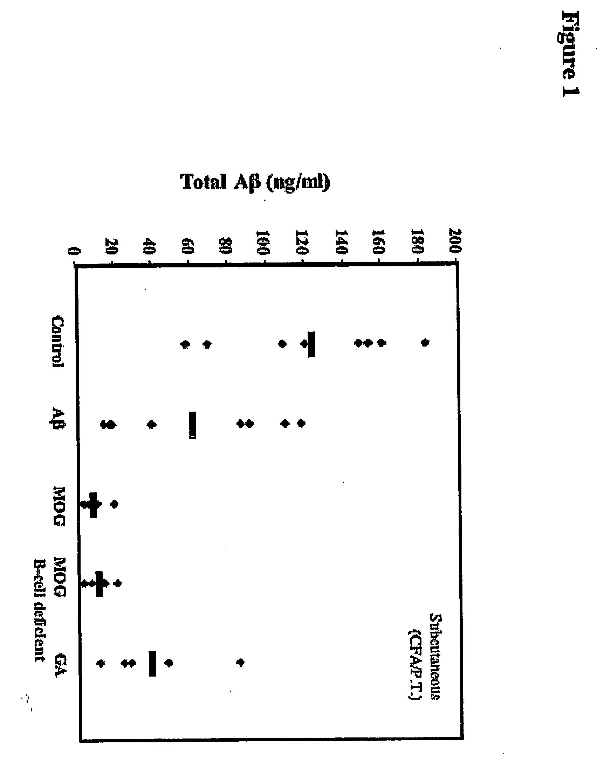

**Figure 1****: Effect of subcutaneous immunization on total Aβ levels in the brain.** To quantify amyloid burden, the right hemisphere was extracted in 5.0 M guanidinium-chloride (pH 8) for 3 hours at room temperature. Dilutions were used to measure levels of Aβ40 and Aβ42 by sandwich enzyme-linked immunosorbent assays (ELISA).

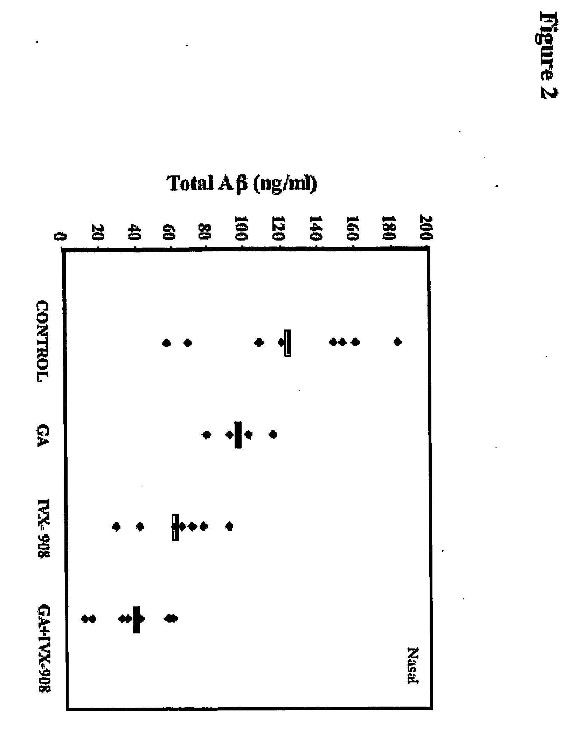

**Figure 2****: Effect of nasal immunization on total Aβ total levels in the brain.** Total Aβ concentration levels of Aβ40 and Aβ42 from individual mice following nasal treatment measured by sandwich enzyme-linked immunosorbent assays (ELISA).

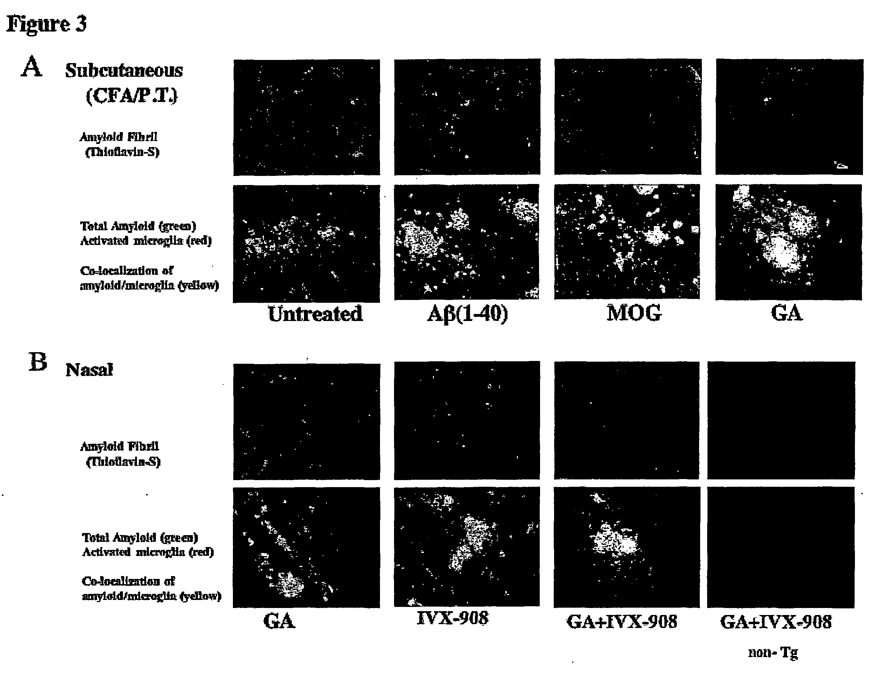

**Figure 3****: Activation of CD11b+ cells lead to clearance of Aβ fibril in parenterally and nasally treated mice.** (A) Staining of Aβ fibril in hippocampal region with thioflavin-S (Magnificationx10) or co-staining for total Aβ with anti Aβ antibody (R1288) and anti-CD11b (microglia/macrophage) Magnification x40) following subcutaneous immunization. (B) Co-staining anti Aβ antibody (R1288) and anti-CD11b. (microglia/macrophage) (in hippocampal region Magnification x40) following nasal immunization.

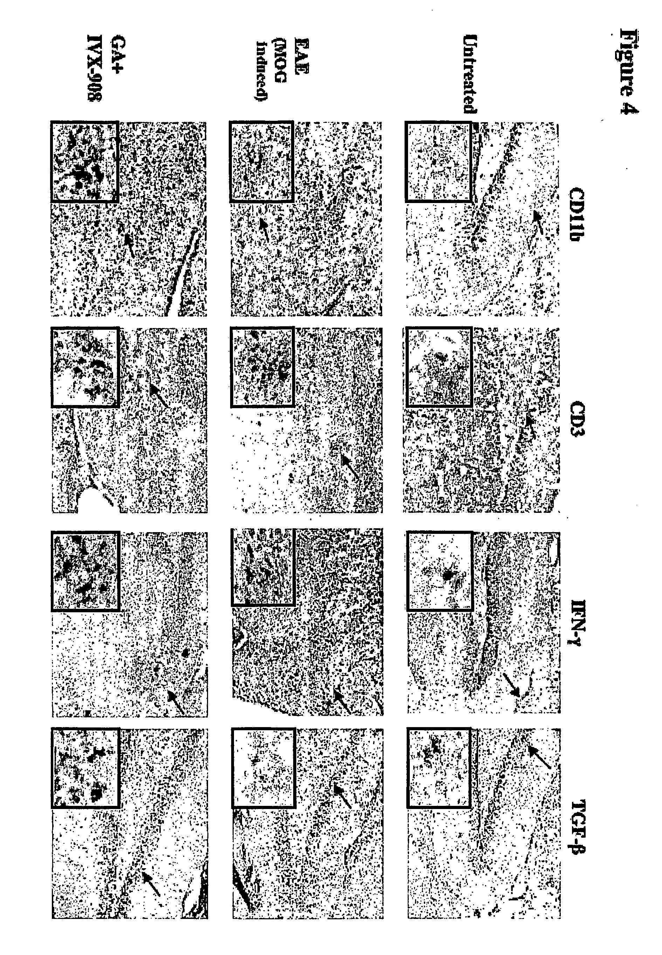

**Figure 4****: Immunohistology of brain sections following MOG subcutaneous immunization and nasal glatiramer acetate vaccination. Serial sections of the** hippocampus region from untreated, or immunized mice 50 days post immunization were labeled using anti CD1 1b, CD3, IFN-γ..and TGF-β antibodies (magnification x20, insert figure magnification x60).

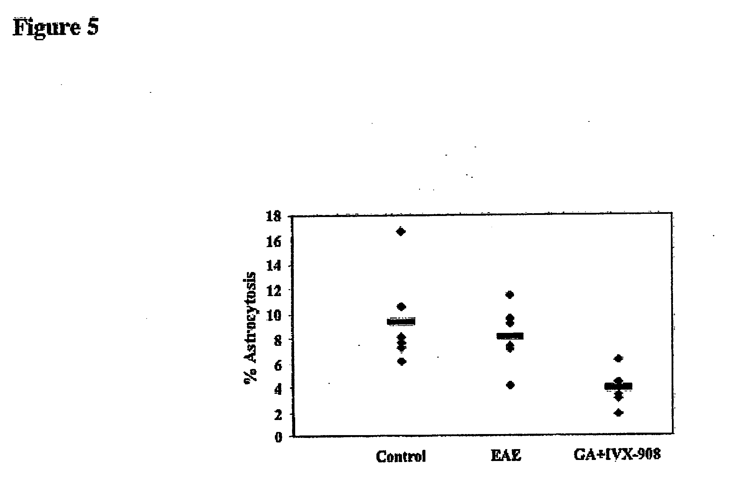

**Figure 5****: Reduction in astrocytosis following nasal administration of GA+IVX-908.** Well-defined hippocampal regions (Bregma -1.44mm), were selected for quantification of activated astrocytes using GFAP staining. The level of astrocyte activation was expressed as a percentage per mm² hippocampal region; p=0.039 GA+IVX-908 vs. control; p=0.02 vs. EAE (MOG).

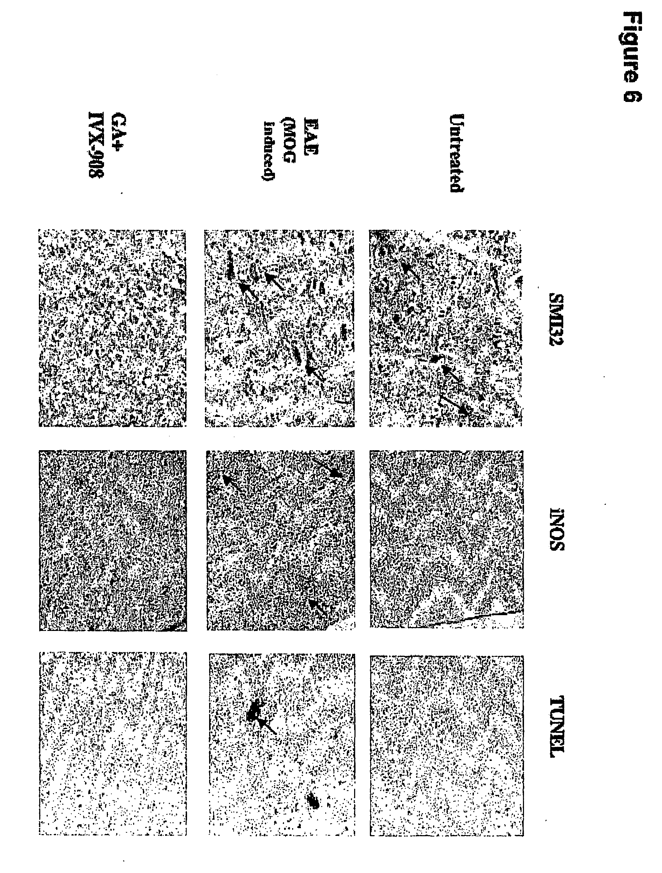

**Figure 6****: Neuropathology in brain sections following MOG subcutaneous immunization and nasal glatiramer acetate vaccination.** Serial sections of the cortex from untreated, or treated mice 50 days post immunization were labeled using markers of neurotoxicty: SMI32, TUNEL, and iNOS (original magnification x20). Arrows identify labeling for markers studied. Labeling for markers of neurotoxicity was observed in EAE animals, but not in GA-IVX-908 treated animals.

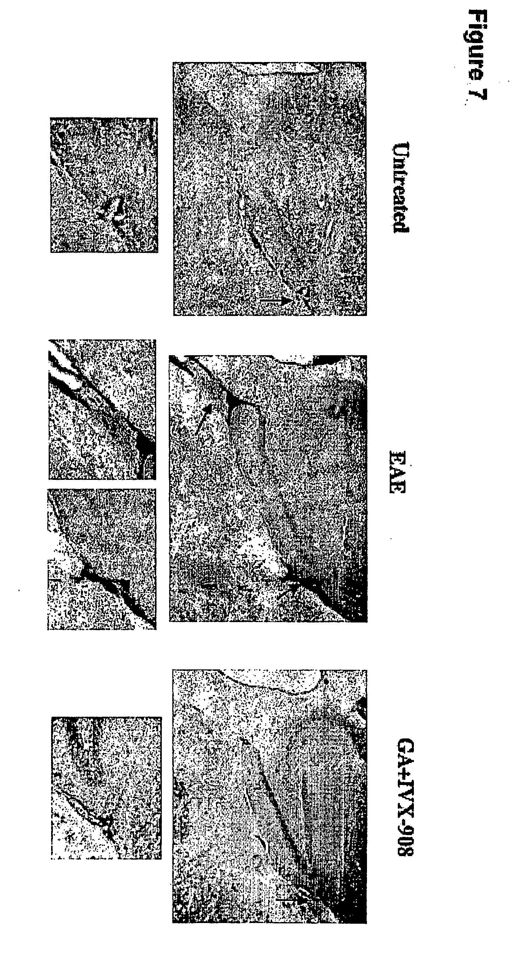

**Figure 7****: Blood brain barrier integrity in hippocampus section following MOG subcutaneous immunization and nasal glatiramer acetate vaccination.** Serial sections of the cortex from untreated, or treated mice 50 days post immunization were labeled using marker of plasma staining -fibrinogen. Labeling for markers of fibrinogen was observed in EAE animals, but not in GA-IVX-908 treated animals. (Magnification x20, small figure magnification x40).

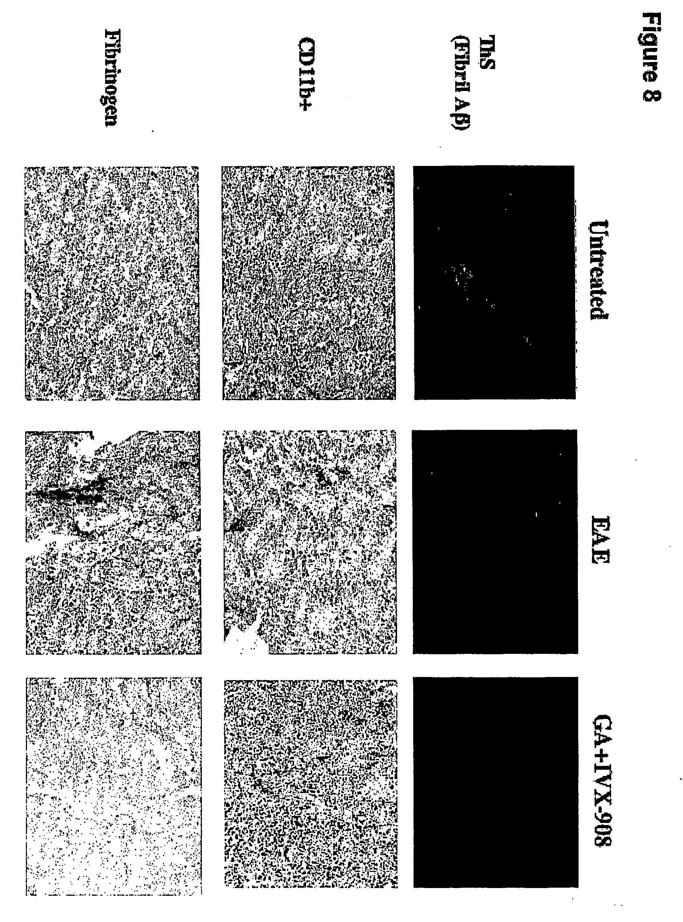

**Figure 8****: Neuropathology in olfactory sections following MOG subcutaneous immunization and nasal glatiramer acetate vaccination.** Serial sections of the cortex from untreated, or treated mice 50 days post immunication were labeled using markers of fibril amyloid: ThS, microglia activation CD11b, BBB integrity, Fibrinogen. (Magnification x20)

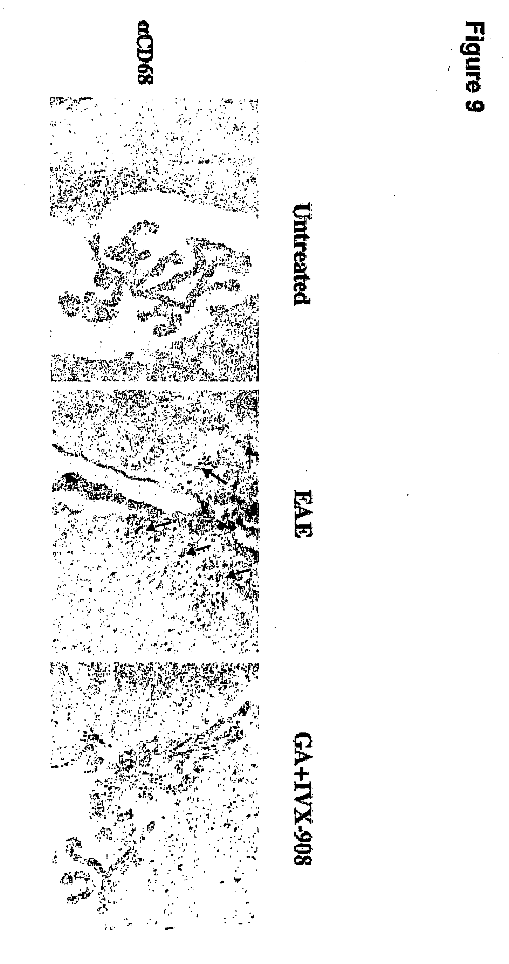

**Figure 9****: Staining for CD68+ cells in the CNS in untreated, MOG immunized and GA+IVX-908 treated mice.** Arrows identify CD68+cells which infiltrate the CNS in EAE, but remain localized to choroids plexus in GA+IVX-908 treated mice. No staining was observed in untreated mice. Sections are taken from the cerebellum (Magnification x20).

### MODES OF PRACTICING THE INVENTION

The term "neurological disease" refers to a disease or disorder, which involves the neuronal cells of the nervous system. Specifically included are: prion diseases (e.g, Creutzfeldt-Jakob disease); pathologies of the developing brain (e.g., congenital defects in amino acid metabolism, such as argininosuccinicaciduria, cystathioninuria, histidinemia, homocystinuria, hyperammonemia, phenylketonuria, tyrosinemia, and fragile X syndrome); pathologies of the mature brain (e.g., neurofibromatosis, Huntington's disease, depression, amyotrophic lateral sclerosis, Multiple Sclerosis); conditions that strike in adulthood (e.g. Alzheimer's disease, Creutzfeldt-Jakob disease, Lewy body disease, Parkinson's disease, Pick's disease); and other pathologies of the brain (e.g., brain mishaps, brain injury, coma, infections by various agents, dietary deficiencies, stroke, multiple infarct dementia, and cardiovascular accidents).

The preferred diseases or disorders of the current invention are those diseases affecting the mature brain, such as Multiple Sclerosis, and those which typically strike in adulthood, such as Alzheimer's disease.

The term "Alzheimer's disease", abbreviated herein as "AD" refers to a neurodegenerative disease of the central nervous system. Broadly speaking, the disease falls into two categories: late onset, which occurs in old age (typically above 65 years) and early onset, which develops well before the senile period, e.g., between 35 and 60 years. In both types of the disease, the pathology is similar, but the abnormalities tend to be more severe and widespread in cases beginning at an earlier age. AD is characterized by the accumulation of extracellularly localized brain amyloid (e.g., Aβ peptide), amyloid plaques (which may be further distinguished as dense or diffuse) and intracellularly localized neurofibrillary tangles concentrated in certain vulnerable regions of the brain such as the hippocampus and cortex. AD is a progressive disease resulting in senile dementia. Amyloid plaques are areas of disorganized neurofibrillary fibers which may be associated with neutrophils up to 150 mm across with extracellular amyloid-beta (Aβ) deposits at the center, visible by microscopic analysis of sections of brain tissue. Neurofibrillary tangles are intracellular deposits of tau protein (often hyperphosphorylated) consisting of two filaments twisted about each other in pairs. AD is associated with the abnormal accumulation of Aβ peptide resulting from altered proteolytic processing of amyloid precursor protein (APP). Abnormal accumulation of Aβ has been correlated with a variety of mutations, such as autosomal dominant APP mutations and mutations in genes encoding proteins referred to as presenilin 1 (PS1) and presenilin 2 (PS2), which subsequently influence the proteolytic activity of γ (gamma), or β (beta) secretase, causing increased levels of, for example, Aβ1-42; whereas, the proteolytic activity of α (alpha) secretase is presumably associated with the normal processing of APP. Various types of plaques are found in AD, including, but not limited to neuritic plaques associated with abnormal dystrophic neurites. Also characteristic of the disease is the presence of an inflammatory response in the CNS, including activated microglia and astrocytes. The accumulation of aggregates of dysfunctional protein (*e.g*., Aβ and prion protein) associated with neurological disorders are thought to cause or contribute to or otherwise influence the development of certain neurological disorders (Ingelsson, M. and Hyman B.T. (2002) Annals of Med. 34:259-271). Neurodegenerative disorders associated with deleterious protein aggregation include Alzheimer's disease, Pick's disease, Parkinson's disease, prion disease, Huntington and motor neuron disorders. (Shastry, Neurochemistry International, 2002, 43:1-7).

The term "amyloid" refers to the extracellular (*e.g*., Aβ, prion diseases, and multiple myeloma light chain disease) or intracellular (*e.g*., neurofibrillary tangles of tau protein in AD and alpha-synuclein in Parkinson's disease) deposition of protein aggregates (Trojanowski J.Q. and Mathson M.P. (2003) Neuromolecular Medicine 4:1-5). Amyloid deposition can be found in the brain of AD and Down's Syndrome patients as well as in arteries, arterioles, capillaries and veins of the central nervous system. Amyloid deposits can be recognized by the ability to bind dyes such as Congo red and thioflavin S, and form fibrils, including a cross β-pleated sheet confirmation.

The term "amyloidosis" refers to a large heterogeneous group of disorders characterized by aberrant insoluble deposits of normally soluble proteins, which may be misfolded proteins, including protein aggregates.

In addition to Alzheimer's disease (AD), early onset Alzheimer's disease, late onset Alzheimer's disease, and presymptomatic Alzheimer's disease, other diseases characterized by amyloid deposits are, for example, Serum Amyloid A (SAA) amyloidosis, hereditary Icelandic syndrome, multiple myeloma, prion diseases and the like, or other brain amylogenic diseases (Revesz, T. et al. (2003) J. Neuropathol. Exp. Neurol. 62(9):885-98), may be treated according to compositions and methods set forth herein. The most common prion diseases in animals are scrapie of sheep and goats and bovine spongiform encephalopathy (BSE) of cattle (Wilesmith and Wells(1991) Curr. Top. Microbiol. Immunol. 172:22-38). Four prion diseases have been identified in humans: (i) kuru, (ii) Creutzfeldt-Jakob disease (CJD), (iii) Gerstmann-Streussler-Sheinker disease (GSS), and (iv) fatal familial insomnia (FFI) (Gajdusek, D.C. (1977) Science 197(4307):943-60 and Medori, R. et al., (1992) N. Engl. J. Med. 326(7):444-9).

The principal constituent of the senile plaques is the Aβ peptide. The Aβ peptide is an internal fragment of 39-43 amino acids of precursor protein APP. Several mutations within the APP protein have been correlated with the presence of Alzheimer's disease (See, e.g., Goate, A. et al., (1991) Nature 349(6311):704-6, Murrell, M. et al., (1991) Science 254(5028):97-9, Mullan, M. et al., (1992) Nat. Genet. 1(5):345-7).

The term "β-amyloid precursor protein" (APP) as used herein is defined as a polypeptide that is encoded by a gene of the same name localized in humans on the long arm of chromosome 21 and that includes Aβ within its carboxyl third. APP is a glycosylated, single-membrane-spanning protein expressed in a wide variety of cells in many mammalian tissues.

APP mutations are thought to influence the development of Alzheimer's disease by increased or altered proteolytic processing of APP to Aβ, particularly processing of APP to increased amounts of the long form of Aβ *(i.e.,* Aβ1-42 and Aβ1-43). Mutations in other genes, such as the presenilin genes, PS1 and PS2, are thought indirectly to affect proteolytic processing of APP to generate increased amounts of long form Aβ (see Hardy, J. (1997) Trends Neurosci. 20(4):154-9). These observations indicate that Aβ, and particularly its long form, is a causative element in Alzheimer's disease.

The term "APP fragments" as used herein refers to fragments of APP other than those which consist solely of Aβ or Aβ fragments. That is, APP fragments will include amino acid sequences of APP in addition to those which form intact Aβ or a fragment of Aβ.

The terms "beta-amyloid peptide" is synonymous with "β-amyloid peptide", "βAP", "βA", and "Aβ". All of these terms refer to a plaque forming peptide derived from fragments of amyloid precursor protein.

As used herein, the definition of the terms fibrillar Aβ and total Aβ are as follows. "Fibrillar" Aβ is Aβ peptide contained in extracellular amyloid deposits which may also be referred to as Aβ plaques or plagues; in some cases, Aβ plaques may be further distinguished as diffuse or dense. "Total" amyloid load or total Aβ load is the sum of soluble and non-soluble (e.g., fibrillar) Aβ peptide, most of which is presumed to be extracellular. It is appreciated that there is a dynamic relationship between soluble and non-soluble Aβ, where, extracellular non-fibrillar Aβ may represent a source of Aβ which may become fibrillar amyloid.

As used herein, the term Experimental Allergic Encephalomyelitis (EAE) is the primary animal model for MS. EAE can readily be induced in small mammals by immunization with MBP in an appropriate adjuvant or by passive transfer of CD4⁺, MBP-reactive T-cells (Alvord Jr, E. C., et al. eds. in Experimental Allergic Encephalomyelitis a Useful Model for Multiple Sclerosis, A. R Liss, N.Y., 1984; Makhtarian et al. Nature 309: 356 (1984); Ben-Nun et al. J. Immunol. 129:303 (1982)). The T-cells that induce EAE in both mice and rats recognize peptides corresponding to immunodominant regions of MBP presented by antigen-presenting cells on class II Major Histocompatibility Complex (MHC) molecules.

According to one aspect of the present invention there is provided a method of treating a neurological disease or disorder in a mammal. The method of this aspect of the present invention may be made effective by administering a therapeutically effective amount of GA and a proteosome based composition to a subject in need thereof. A further aspect of the present invention is wherein said neurological disease or disorder is a amyloid plaque-forming disease or disorder. The most prominent neurological diseases or disorders treated with GA and a proteosome based composition according to one aspect of the present invention is Alzheimer's disease and Multiple Sclerosis. In addition to a therapeutic composition of the GA combined with a proteosome for treatment of the aforementioned neurological disease or disorders, further embodiments include, but are not limited to, using the following therapeutic compositions for treatment: a proteosome based composition without a GA composition, GA in a submicron emulsion composition, or GA in a nanoemulsion composition. The previous embodiments would also include any pharmaceutically acceptable diluent, excipient, stabilizer or carrier.

A yet further aspect of the present invention is wherein the treatment of said neurological disease or disorder in a mammal comprises administering a therapeutically effective amount of GA and a proteosome based composition which elicits an antibody independent response in said mammal.

A further embodiment of the present invention consists of a method for treating an amyloidal disease, which may be Alzheimer's disease. The treatment of amyloidal disease may be carried out by, for example, preventing an increase in fibrillar amyloid load, preventing an increase in total amyloid load, maintaining the current fibrillar and/or total amyloid load, or decreasing the fibrillar and/or total amyloid load in the brain.. As it is known that amyloidal proteins may reside throughout the body, embodiments of the present invention are not limited to brain amyloid. Furthermore, although the present invention specifically discusses β-amyloid, other amyloid classes such as serum amyloid A (SAA), prion disease, hereditary Icelandic syndrome, Huntington disease, Parkinsonism, Down's Syndrome and cerebral amyloid angiopathy are considered within the current invention. According to the aforementioned amyloidal diseases, treatment may be accomplished by administering one of the following therapeutic compositions: a therapeutically effective amount of GA and a proteosome based composition, a proteosome based composition without a GA composition, GA in a submicron emulsion, and/or GA in a nanoemulsion. The previous embodiments would also include any pharmaceutically acceptable diluent, excipient, stabilizer or carrier.

### GLATIRAMER ACETATE

An immunochemical analog of MBP that is effective in treating multiple sclerosis (MS) is glatiramer acetate (GA), or copolymer-1 (Cop-1) (U.S. Pat. No. 3,849,550; PCT Application WO/95/31990). GA, in its commercially available form, is a mixture of random synthetic polypeptides composed of L-alanine, L-glutamic acid, L-lysine and L-tyrosine in a molar ratio of 6.0:1.9:4.7:1.0. It was first synthesized as an immunochemical mimic of MBP. For example, certain monoclonal antibodies to GA cross-react with MBP (Teitelbaum et al. Proc. Natl. Acad. Sci. USA 88:9258 (1991)). Also, GA has been found to induce T suppressor cells specific for MBP (Lando et al. J. Immunol. 123:2156 (1979)). Experiments in mice indicate that GA also specifically inhibits MBP-specific T cells that are involved in the destruction of central nervous system tissue in EAE (Teitelbaum et al. Proc. Natl. Acad. USA 85:9724 (1995); and Angelov, D.N. et al. PNAS 100(8):4790-4795 (2003)), indicate that GA can be used to treat amyotrophic lateral sclerosis, where induction of a well-regulated autoimmune response appears to influence survival in the presence of an anti-self T-cell response, which may be enhanced by administration of GA.

GA, according to the present invention, may be prepared by methods known in the art. For example, GA may be prepared by the process disclosed in U.S. Pat. No. 3,849,550, wherein the N-carboxyanhydrides of tyrosine, alanine, γ-benzyl glutamate and ∈-N-trifluoro-acetyllysine are polymerized at ambient temperature in anhydrous dioxane with diethylamine as an inhibitor. The deblocking of the γ-carboxyl group of the glutamic acids is carried out with hydrogen bromide in glacial acetic acid and is followed by the removal of the trifluoracetyl groups from the lysine residues by 1M piperidine. The resulting mixture of polypeptides consists essentially of polymers of alanine, glutamic acid, lysine, and tyrosine, in a molar ratio of about 6:2:5:1.

GA is also available commercially from Teva Pharmaceuticals, Kfar-Saba, Israel.

GA may be prepared for use according to the instant invention in any of the forms which maintain its therapeutic utility. These include mixtures of peptides having various molecular weight ranges. GA having a desired molecular weight range can be obtained by methods known in the art. Such methods include gel filtration high pressure liquid chromatography of GA to remove high molecular weight species as disclosed in WO 95/31990. In one embodiment, the GA has about 75% of its polymer species within the molecular weight range of about 2 KDa to about 20 KDa. In another embodiment, GA has an average molecular weight from about 4 KDa to 9 KDa. It is understood that GA may be subjected to enzymatic or other degradation in order to comprise polymer species of a length different from, or otherwise modified, from conventional GA according to the known methods.

### GA AND PROTEOSOMES

GA formulated with proteosomes (e.g., IVX-908 or Protollin) may be administered by, for example, injection or intranasally. When delivered by injection, such delivery may be as one injection (combined simultaneous administration). Alternatively, delivery of a GA composition and a proteosome based composition may be delivered separately, accomplished by injection at a plurality of sites which may occur simultaneously or at temporally distinct times and where one site is presented only with a GA composition (*i.e*., no proteosomes) and, at a second site only a proteosome based composition is administered (*i.e*., no GA). It is also contemplated that GA may be administered by injection while at the same or different time a proteosome based composition is administered, for example, intranasally. Thus, in one embodiment of the instant invention, a GA composition is delivered by injection and separately, a proteosome based composition is delivered intranasally.

GA peptides of the instant invention may also be prepared to contain a hydrophobic anchor sequence moiety which would be expected to enhance non-covalent association with proteosomes. The production and manufacture of proteosome-amphiphilic determinant vaccines designed for either parenteral or especially for mucosal administration including, gastro-intestinal administration to induce both systemic and mucosal antibody responses is discussed in U.S. Pat No. 6,476,201. An amphiphilic determinant is a molecule having hydrophobic and hydrophilic regions which, when appropriately formulated with proteosomes, align with the proteosomes to form a complex which elicits an immunologic response in a subject. Typical amphiphilic determinants include glycolipids, liposaccharides (including detoxified lipopolysaccharides), lipopeptides, transmembrane domains, envelope or toxoided proteins, or proteins or peptides with intrinsic hydrophobic amino acid anchors.

### EMULSION COMPOSITIONS

GA can be administered in or formulated with a submicron emulsion or nanoemulsion as described in US Patent 5,961,970 or 5,716,637, respectively. The composition comprises an oil-in -water submicron emulsion with about 0.5 to about 50% of an oil, about 0.1 to about 10% of an emulsifier, about 0.05 to about 5% of a nonionic surfactant, and about 0.00001 to about 1.0% of GA as an aqueous continuous phase. The submicron emulsion has a mean droplet size in the range of between about 0.03 and about 0.5 µm, and preferably 0.05 and 0.2 µm.

The nanoemulsion approach provides vaccine compositions containing nanoemulsions of particles having a lipid core, surrounded by at least one phospholipid bilayer. The particles have a mean diameter in the range of about 10 to about 250 nm, as determined on a weight basis, and the GA is incorporated therein, either intrinsically prior to the homogenization process or extrinsically thereafter. The particles are typically suspended in an aqueous continuous phase and each lipid particle comprises a lipid core, wherein the lipid is a solid or liquid crystal in bulk at a temperature of about 25°C or higher. Usually the amount of GA entrapped is about 0.001 to about 5%. Alternatively, the GA can be formulated with a proteosome and/or the nanoemulsion can further comprise a bioadhesive or mucoadhesive macromolecule.

### PROPOSED MECHANISM

The invention also includes, for example, a method for inhibiting or reducing the level of an amyloid-β peptide (soluble and/or non-soluble), fragment or derivative thereof, which comprises immunizing a mammal with GA formulated with a proteosome based composition, or a proteosome based composition without GA, wherein the reduction or inhibition of the peptide, fragment or derivative thereof occurs without the generation of antibodies, as demonstrated herein using B-cell deficient (µMT) mice, which are incapable of eliciting an antibody response. Although not wishing to be bound by theory, preferably, the inhibition or reduction of amyloid (e.g., Aβ peptide) is via an activation of immune cells such as brain localized microglia, or neutrophils and/or macrophages which may be found in the brain or peripherally, wherein the activation of these cells is independent of any antibody or antigen-specific mechanism. It is most preferred that the reduction or inhibition occurs without causing Experimental Allergic Encephalomyelitis (EAE) in the mammal (including meningoencephalitis).

The reduction of amyloid load (e.g., Aβ comprising fibrillar plaques plus non-fibrillar Aβ that may not be deposited into plaques) relates to the reduction of amyloid deposition and/or Aβ plaque formation and thereby the treatment of AD and related diseases or amyloidogenic diseases or disorders. Although the various aspects of the invention should not be limited to any particular theory or mechanism, it is believed that activated microglia co-localize with amyloid plaques and the activation of microglial cells is dependent upon the presence of amyloid deposition, which deposition primes the endogenous microglial cells for activation. Thus, activated microglial cells participate in clearing amyloid (e.g., Aβ plaque) deposits. Additional information on the possible mechanisms for AD is found in Schenk, D. (2002) Nature 3:824-828. Deposition of Aβ and formation of amyloid plaques appears to be accompanied by a complex inflammatory and neurotoxic cascade. Therefore, it is thought that an anti-inflammatory mechanism of treatment may be beneficial. Such anti-inflammatory processes are often consistent with expression of anti-inflammatory IL-4/IL-10 (Th2) and TGF-β (Th3) immune responses. Consequently it is a surprising and unexpected finding that the instant formulations comprising proteosome based compositions and GA stimulate a non-antibody mediated immune response that causes the reduction of Aβ-amyloid containing plaques, as it has previously been proposed that proteosomes are more often associated with the stimulation of a Th1 type immune (cytokine) response associated with a pro-inflammatory immune response and would be appositive to and distinct from a Th2 immune response. Nevertheless, augmentation of microglial phagocytosis by the Th1-type cytokine INF-gamma (75% above untreated), might also suggest a feedback mechanism for accelerated clearance of the inflammatory infiltrate in the CNS (Cha, A. et. al. (2001) GLIA 33(1):87-95). Furthermore, INF-gamma by itself can also lead to the transcriptional inhibition of the beta-amyloid precursor protein (Ringheeim G. E. et al. Biochem Biophys Res Commun (1996) 224(1):246-51).

### PROTEOSOME BASED COMPOSITIONS

The subject of US Patents 6,476,201 and 5,961,970 describes how in order for multivalent subunit vaccines to stimulate optimal immune responses to each of the components, the proper components should be appropriately associated and each be available to the immune system so that they may be efficiently recognized and processed by cells of the immune system. Such recognition and processing may include uptake by membranous cells (M-cells) located within, for example, the nasal epithelia and subsequent delivery to underlying cells of the host immune system. Prime examples of such non-covalently complexed vaccines include proteosome based vaccines which can consist of *Neisserial* outer membrane proteins non-covalently complexed to a wide variety of antigens including peptides, lipopeptides, transmembrane or toxoided proteins, polysaccharides or lipopolysaccharides (LPS) (for further review see the following references; US Patent 5,726,292, Immunogenicity and Efficacy of Oral or Intranasal *Shigella flexneri* 2a and *Shigella sonnei* Proteosome-Lipopolysaccharide Vaccines in Animal Models; Infect. Immun. 61:2390; Mallett, C. P., T. L. Hale, R. Kaminski, T. Larsen, N. Orr, D. Cohen, and G. H. Lowell. (1995)); Intranasal or intragastric immunization with proteosome-Shigella lipopolysaccharide vaccines protect against lethal pneumonia in a murine model of shigellosis (Infect. Immun. 63:2382-2386; Lowell G H, Kaminski R W, Grate S et al. (1996)); Intranasal and intramuscular proteosome-staphylococcal enterotoxin B (SEB) toxoid vaccines: immunogenicity and efficacy against lethal SEB intoxication in mice (Infec. Immun. 64:1706-1713; Lowell, G. H. (1990)); Proteosomes, Hydrophobic Anchors, Iscoms and Liposomes for Improved Presentation of Peptide and Protein Vaccines, (in New Generation Vaccines: G. C. Woodrow and M. M. Levine, eds. (Marcel Dekker, NY) Chapter 12 (pp. 141-160)); and Proteosome-lipopeptide vaccines: enhancement of immunogenicity for malaria CS peptides; (Lowell, G. H., W. R. Ballou, L. F. Smith, R. A. Wirtz, W. D. Zollinger and W. T. Hockmeyer (1988) Science 240:800)).

Proteosome based compositions as used herein refers to preparations of outer membrane proteins (OMPs, also known as porins) from Gram-negative bacteria, such as *Neisseria* species (see, e.g., Lowell et al., J. Exp. Med. 167:658, 1988; Lowell et al., Science 240:800, 1988; Lynch et al., Biophys. J. 45:104, 1984; Lowell, in "New Generation Vaccines" 2nd ed., Marcel Dekker, Inc., New York, Basil, Hong Kong, pages 193, 1997; U.S. Patent No. 5,726,292; U.S. Patent No. 4,707,543), which are useful as a carrier or an adjuvant for immunogens, such as bacterial or viral antigens. Proteosomes prepared as described above also contain an endogenous lipopolysaccharide (LPS) originating from the bacteria used to produce the OMP porins *(e.g., Neisseria* species). Any preparation method that results in the outer membrane protein component in vesicular or vesicle-like form, including multi-molecular membranous structures or molten globular-like OMP compositions of one or more OMPs, is included within the definition of proteosome.

"Liposaccharide" as used herein refers to native or modified lipopolysaccharide or lipooligosaccharide (collectively, also referred to as "LPS") derived from Gram-negative bacteria such as *Shigella flexneri* or *Plesiomonas shigelloides,* or other Gram-negative bacteria (including *Alcaligenes, Bacteroides, Bordetella, Brucella, Campylobacter, Chlamydia, Citrobacter, Edwardsiella, Ehrlicha, Enterobacter, Escherichia, Francisella, Fusobacterium, Gardnerella, Hemophillus, Helicobacter, Klebsiella, Legionella, Moraxella, Morganella, Neiserria, Pasteurella, Proteus, Providencia,* other *Plesiomonas, Porphyromonas, Prevotella, Pseudomonas, Rickettsia, Salmonella, Serratia,* other *Shigella, Spirillum, Veillonella, Vibrio,* or *Yersinia* species). It should be noted that LPS as used herein may be non-detoxified or detoxified.

In other embodiments exogenous LPS isolated from the same or different bacteria from which a proteosome was prepared may be mixed therewith; one such proteosome based composition is referred to herein as IVX-908 (may also be referred to as Protollin). In other words, proteosomes of the IVX-908 type are preparations of OMPs admixed with at least one kind of liposaccharide to provide an OMP-LPS composition (which can function as an immunostimulatory composition). Thus, the OMP-LPS (IVX-908) adjuvant can be comprised of two of the basic components (1) an outer membrane protein preparation of proteosomes prepared from Gram-negative bacteria, such as *Neisseria meningitides,* and (2) a preparation of one or more liposaccharides. It is also contemplated that components of IVX-908 may be or include lipids, glycolipids, glycoproteins, small molecules, or the like.

Proteosome based compositions as disclosed herein may include one or more components which, at least in part, function as an adjuvant possessing the capacity to stimulate a host immune system. It is appreciated that such proteosome components may include an outer membrane protein (OMP) component (fusion protein or fragment thereof) of gram negative bacteria as well as a lipopolysaccharide (LPS) component of a same or different gram negative bacteria. Such components may function as, for example, ligands which stimulate a host immune response by interacting with certain receptors (*e.g*., Toll-like receptors) produced by one or more host cells of a vaccine recipient.

Without wishing to be bound by theory, one or more components of vaccine formulations disclosed herein may interact with Toll-like receptors (TLRs) associated with an innate and/or adaptive immune response of a vaccine recipient. There are at least 10 TLRs (see Takeda et. al., Annu Rev Immunology (2003) 21:335-76). One or more ligands which interact with and subsequently activate certain TLRs have been identified, with the exception of TLR8 and TLR10. Outer membrane proteins of *Neisseria meningitides,* for example OMP 2 (also referred to as Por B), interacts with TLR2, while LPS of most but not all gram negative bacteria interacts with TLR4. Accordingly, one mechanism by which vaccine formulations described herein may contribute to a biological affect includes activation of one or both of TLR2 and TLR4. However, in another aspect of the instant invention, it is equally possible that activation of other TLRs (other than TLR2 and TLR4) may serve a similar function or further enhance the qualitative and/or quantitative profile of cytokines expressed, and which may be associated with a host Th1/Th2 immune response.

The qualitative and/or quantitative activation of one or more TLRs is expected to elicit or influence a relative stimulation (balanced or imbalanced) of a Th1 and/or Th2 immune response, with or without concomitant humoral antibody response.

Ligands interacting with TLRs other than TLR2 and TLR4 may also be used in vaccine compositions described herein. Such vaccine components may, alone or in combination, be used to influence the development of a host immune response sufficient to treat or protect from amylogenic disease, as set forth herein. Such TLRs and associated ligands include, but are not limited to, those presented in List 1.

### List 1

| **TLR family** | **Ligands** |
|---|---|
| | (example of possible source) |
| TLR1 | Soluble factors (*Neisseria meningitides*) Tri-acyl lipopeptides (bacteria, mycobacteria) |
| TLR2 | Lipoproteins and lipopeptides |
| | Porins *(Neisseria)* |
| | Atypical LPS *(Leptospira interrogans)* |
| | Atypical LPS *(Porphyromonas gingivalis)* |
| | Peptidoglycan (Gram-positive bacteria) |
| | Lipoteichoic acid (Gram-positive bacteria) |
| | HSP70 (host) |
| | Glycolipids *(Treponema maltophilum)* |
| TLR3 | Double-stranded RNA (*e.g*., viral) |
| TLR4 | LPS (Gram-negative bacteria) |
| | Taxol (plant) |
| | HSP60 (host) |
| | HSP70 (host) |
| | HSP60 (*Chlamydia pnemoniae*) |
| | Fibrinogen (host) |
| TLR5 | Flagellin (bacteria) |
| TLR6 | Di-acyl lipopeptides (mycoplasma) |
| TLR7 | Imidazoquinoline (synthetic compounds) |
| | Loxoribine (synthetic compounds) |
| | Bropirimine (synthetic compounds) |
| TLR8 | Ligand yet to be identified |
| TLR9 | CpG DNA (bacteria) |
| TLR10 | Ligand yet to be identified |

Any one or combination of the identified TLRs (List 1) may be activated by any one or combination of TLR ligand components of a vaccine formulation contemplated herein. It is further appreciated that stimulation of any one or multiplicity of TLRs may be accomplished using any one or a multiplicity of TLR ligands at concentrations suitable with the route of administration (*e.g*., intranasal, injection etc.).

Therefore, according to the instant invention, it is understood that a vaccine formulation may include any one or more TLR ligand(s), including recombinant ligands (fusion proteins or fragments thereof) combined with an antigenic vaccine component, or optionally including CD14 receptor, with or without exogenous addition of a lipopolysaccharide component.

In one aspect of the instant invention only the TLR binding portion of any one or more TLR ligands may be isolated by, for example, recombinant DNA technologies and formulated with or without GA as a therapeutic treatment and/or prophylactic prevention of Alzheimer's Disease or similar disease or disorder. Such a polypeptide may also be prepared by one or another synthetic procedures well known to those of skill in the art. Not wishing to be bound by theory, one such isolated binding domain may be isolated from a portion of *Neisseria meningitidis* outer membrane protein referred to as Porin B which is suspected of binding to TLR2. Other such polypeptide ligand binding domains of TLRs may also be used in similar fashion alone or in one or another combination. Such a formulation may be used with or without the necessity of administering a Proteosome-GA formulation, or may be used subsequent to administration of a Proteosome-GA formulation. In addition, it is appreciated that a variant (e.g., conservative amino acid substitution) of such a TLR binding portion of a TLR ligand maybe used to activate a TLR as long as the variant maintains the ability to bind (and activate) the TLR. In still a further aspect of the instant invention, such a TLR binding portion of a TLR ligand may be reiterated one or more times using recombinant DNA technologies to prepare a polypeptide containing multiple copies of such binding portion, or even multivalent (i.e., hybrid) polypeptides comprising multiple binding domains of the same or different TLR ligands.

In certain proteosome based compositions, one or more of the component parts of the vaccine formulation need not be non-covalently complexed but rather may be mixed with the proteosome composition (e.g., IVX-908, Protollin). Proteosome based compositions termed Projuvant contain only small amounts of endogenous LPS (or lipooligosaccharide (LOS)) while IVX-908/protollin proteosome based compositions contain additional exogenous LPS which may be from the same or different gram negative bacteria species as the OMP components or may be a mixture of LPS derived from more than one gram negative bacteria.

In one embodiment, the final liposaccharide content by weight as a percentage of the total proteosome protein can range from about 1% to about 500%, more preferably in a range from about 20% to about 200%, or in a range from about 30% to about 150% or in a range of about 10% to about 100%. A preferred embodiment of the instant invention is the immunostimulatory composition wherein the proteosome based component is prepared from *Neisseria meningitides* and the final liposaccharide content is between 50% to 150% of the total proteosome protein by weight. The final LPS content may represent the combination of endogenous LPS (e.g., LOS) plus exogenously added LPS (or LOS). In another embodiment, proteosome based compositions (*e.g*., Projuvant) are prepared with endogenous lipooligosaccharide (LOS) content from *Neiserria* ranging from about 0.5% up to about 5% of total OMP. Another embodiment of the instant invention provides proteosomes with endogenous liposaccharide in a range from about 12% to about 25%, and in a preferred embodiment between about 15% and about 20% of total OMP. The instant invention also provides a composition containing liposaccharide derived from any Gram-negative bacterial species, which may be from the same Gram-negative bacterial species that is the source of proteosomes or from a different bacterial species.

U.S. Patent 6,476,201 relates to the production and manufacture of proteosome-amphiphilic determinant vaccines designed for either parenteral or mucosal administration to induce both systemic (serum) and mucosal (including respiratory and intestinal) antibody responses. Mucosal administration is preferred, and includes, but is not limited to, respiratory (e.g. including intranasal, intrapharyngeal and intrapulmonary), gastro-intestinal (e.g. including oral or rectal) or topical (e.g. conjunctival or otic) administration. An amphiphilic determinant is a molecule having hydrophobic and hydrophilic regions which, when appropriately formulated with proteosomes, align with the proteosomes to form a complex which elicits an immunologic response in a subject. Typical amphiphilic determinants include glycolipids, liposaccharides (including detoxified lipopolysaccharides), lipopeptides, transmembrane, envelope or toxoided proteins, or proteins or peptides with intrinsic hydrophobic amino acid anchors. These determinant materials can be obtained from gram negative bacteria including *Escherichia, Klebsiella, Pseudomonas, Hemophilus, Brucella, Shigella* and *Neisseria.* More specifically, the proteosome vaccines in which meningococcal outer membrane protein proteosome preparations (prepared from any strain of *N. meningitides* or *N. gonorrhea* or other *Neisserial* species) are non-covalently complexed to native or detoxified *Shigella* or *Neisserial lipopolysaccharides* or lipooligosaccharides to form vaccines are designed to protect against diseases caused by gram negative organisms that contain any of the component parts of the complex including *Meningococci* or *Shigellae.* More specifically, the proteosome vaccines contain LPS that induce antibody responses that recognize type-specific somatic polysaccharide O-antigens of *Shigella* lipopolysaccharides and thereby confer homologous protection against shigellosis. The lipopolysaccharides, when complexed to proteosomes, induce *anti-shigella* protective immune responses. The proteosome vaccines are prepared and purified from either *Shigella sonnei* or *Plesiomonas shigelloides* for immunity against *Shigella sonnei* disease, from *Shigella flexneri* 2a for immunity to *Shigella flexneri* 2a disease, and so forth, using LPS derived from homologous or antigenically cross-reacting organisms to confer homologous immunity against shigellosis caused by *S. flexneri* 2a (or 3a etc.), *S. boydii, S. sonnei* etc. Further, US Patent 6,476,201 describes the administration of proteosome-Shigella vaccines that are multivalent in that two independently made proteosome vaccines using *shigella* LPS antigen derived from *S. flexneri 2a* (for *S. flexneri 2a* disease) and from *P. shigelloides* or *S. sonnei* (for *S. sonnei* disease) are administered together thereby inducing antibodies that recognize the two organisms and thereby conferring protection against the two types of diseases. Furthermore, a proteosome-Shigella LPS vaccine, in which proteosomes from group B type 2b *meningococci* are complexed to *P. shigelloides* LPS using hollow fiber diafiltration technology to produce a vaccine administered by mucosal respiratory and/or gastro-intestinal routes and to induce antibodies that recognize the somatic O-antigen LPS of *S. sonnei,* are thereby used to protect against shigellosis.

An exemplary but non-limiting proteosome based composition of the present invention is proteosome based mucosal adjuvant IVX-908 (Protollin) which is a non-covalent formulation of *Neisseria meningitides* outer membrane proteins (proteosomes) and exogenously added LPS prepared from *Shigella flexneri.*

### FORMULATION AND ADMINISTRATION

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, Pa., latest edition.

Suitable routes of administration may, for example, include oral, rectal, transmucosal, transnasal, intestinal or parenteral delivery, including intramuscular, subcutaneous and intramedullary injections as well as intrathecal, direct intraventricular, intravenous, intraperitoneal, intranasal, or intraocular injections, but the preferred route of administration for proteosomes is intranasally.

GA formulations comprising proteosome based compositions, or in submicron emulsions or nanoemulsions can be administered parenterally, orally, intranasally, or topically, or, as indicated herein, in any combination thereof. Although in certain embodiments it is preferable to administer them parenterally or mucosally.

Dual or multiple routes of administration are also included herein. Dual routes of administration may include, for example, proteosome based preparations prepared and administered intranasally but separately from GA which may be administered by injection at the same time or at a time different from intranasal administration of proteosomes. Proteosome (Projuvant) or proteosome:LPS (i.e., IVX-908) compositions (in the absence of GA) may also be administered by injection simultaneously with GA or at a different time. For injection, the active ingredients of the invention may be formulated in a physiologically acceptable carrier, preferably in physiologically compatible buffers such as Hank's solution, Ringer's solution, or physiological salt buffer. For transdermal and possibly transmucosal administration, penetrants appropriate to the barrier to be permeated may be used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as crosslinked polyvinyl pyrrolidone, agar, or alginic acid or a salt thereof such as sodium alginate.

For nasal administration, the active ingredients for use according to the present invention may be conveniently delivered in the form of, for example, an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges, made of gelatin for example, for use in a dispenser may be formulated to contain a powder mix of the compound and a suitable powder base such as lactose or starch.

The preparations described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

The pharmaceutical compositions of the present invention can be administered for prophylactic and/or therapeutic treatment of diseases related to the production of and/or deposits of amyloid (e.g., Aβ) anywhere in the subject's body, but especially in the brain. In therapeutic applications, the pharmaceutical compositions are administered to a mammal already suffering from the disease and in need of treatment. The pharmaceutical compositions will be administered in an amount sufficient to inhibit or reduce further deposition of Aβ into plaque and/or clear already formed plaque and/or to stimulate removal of already existing Aβ aggregates, and/or stimulate a reduction in Aβ that may not be contained in plaques. An amount adequate to accomplish this is defined as a "therapeutically effective dose or amount."

For prophylactic applications, the pharmaceutical compositions of the present invention are administered to a mammalian subject susceptible to an amyloid related disease (e.g., Aβ related Alzheimer's disease), but not already suffering from such disease. Such mammalian subjects may be identified by genetic screening and clinical analysis, as described in the medical literature (e.g. Goate (1991) Nature 349:704-706). The pharmaceutical compositions will be able to inhibit, prevent or reduce amyloid deposition of, for example, Aβ into plaque at a symptomatically early stage, preferably preventing even the initial stages of the β-amyloid related disease. The amount of the compound required for such prophylactic treatment, referred to as a prophylactically-effective dosage, may, but not necessarily, be generally the same as described above for therapeutic treatment.

For purposes of this specification and the accompanying claims the terms "patient", "subject" and "recipient" are used interchangeably. They include humans and other mammals (e.g., cow and other bovine) which are the object of either prophylactic, experimental, or therapeutic treatment.

As used herein the term "treating" includes substantially inhibiting, slowing or reversing the progression of a disease, substantially ameliorating clinical symptoms of a disease or substantially preventing the appearance of clinical symptoms of a disease, in a statistically significant manner.

Depending on the severity and responsiveness of the condition to be treated, dosing can be of a single or a plurality of administrations at one or a plurality of sites or delivery means, with the course of treatment lasting from several days to several weeks or until cure is effected or a statistically significant diminution of the disease state is achieved. The amount of a treatment to be administered will, of course, be dependent on the subject being treated, the severity of the affliction, the manner of administration, the judgment of the prescribing physician, etc. Methods for calculating statistical significance are known in the relevant art.

"Treatment" of MS is intended to include both treatment to prevent or delay the onset of any manifestation, clinical or subclinical, e.g., histological, symptoms thereof of Multiple Sclerosis, as well as the therapeutic suppression or alleviation of symptoms after their manifestation by abating autoimmune attack and preventing or slowing down autoimmune tissue destruction. "Abatement", "suppression" or "reduction" of autoimmune attack or reaction encompasses partial reduction or amelioration of one or more symptoms of the attack or reaction. A "substantially" increased suppressive effect (or abatement or reduction) of the "autoimmune reaction" means a significant decrease in one or more markers or histological or clinical indicators of MS. Non-limiting examples are a reduction by at least 1 unit in limb paralysis score.

GA is generally administered to treat MS in a dose of 0.01 mg to 1000 mg/day. In one embodiment a dosage in the range of 0.5-50 mg is employed. However, according to one aspect of the instant invention it is anticipated that the use of one or more adjuvants set forth herein (or combination thereof) may be formulated with a composition comprising GA whereby lower or higher doses or frequency of administration of GA may be permitted and that it is not necessary that the dose of GA be effective by itself.

Establishing the effective dosage range as well as the optimum amount is well within the skill in the art in light of the information given in this section. For example, dosages for mammals, and human dosages in particular are optimized by beginning with a relatively low dose of GA (e.g., 1 mg/day), progressively increasing it (e.g., logarithmically) and measuring a biological reaction to the treatment; for example, (i) measuring induction of regulatory cells (CD4⁺ and/or CD8⁺) (Chen, Y. et al., Science, 255: 1237 (1994)); (ii) measuring reduction in class II surface markers on circulating T-cells; (iii) measuring the number of TGF-β expressing cells or the relative amount of detectable TGF-β; (iv) assessing the number and activation of immune attack T-cells in the blood (e.g., by limiting dilution analysis and ability to proliferate); or, (v) by scoring the disease severity, according to well-known scoring methods (e.g., by measuring the number of attacks, joint swelling, grip strength, stiffness, visual acuity, ability to reduce or discontinue medication). An effective dosage is any dose that causes at least a statistically or clinically significant attenuation in one of these markers and preferably one that attenuates at least one symptom characteristic of MS during the dosing study.

Assessment of the disease severity in MS can be accomplished according to well-known methods depending on the type of disease. Such methods include without limitation: severity and number of attacks over a period of time; progressive accumulation of disability (which can be measured, e.g., on the Expanded Disability Status Scale); number and extent of lesions in the brain (as revealed, e.g., by magnetic resonance imaging); and frequency of autoreactive T-cells.

Having now generally described the invention, the same will be more readily understood through reference to the following examples which are provided by way of illustration, and are not intended to be limiting of the present invention, unless specified.

### EXAMPLES

Amyloid precursor protein (APP) transgenic mice were immunized with myelin oligodendrocyte glycoprotein (MOG) peptide (amino acids 35-55) in complete Freund's adjuvant (CFA) with subsequent administration (injection) of pertussis toxin (PT) to determine their susceptibility to Experimental Allergic Encephalomyelitis (EAE) compared to their non-transgenic littermates. As controls, animals were immunized with bovine serum albumin (BSA) or human β-amyloid peptide (Aβ amino acids 1-40). EAE developed to an identical degree in APP transgenic animals (Mucke et al., Ann NY Acad Sci (777) 82-88 (1996)) and their non-transgenic littermates, and no EAE was observed in animals immunized with Aβ 1-40 or with BSA. However, when the brains were examined neuropathologically, there was less staining for Aβ in animals that developed EAE. By quantifying the amount of staining for Aβ fibril in the hippocampus using thioflavin S, a 92% reduction in the MOG-immunized mice was found vs. controls (p=0.001) and a 73% reduction compared to mice immunized with Aβ 1-40 (p=0.03) (See Table 1, Fig 1). By quantifying total brain Aβ content by ELISA, a 94% reduction in Aβ in MOG-immunized animal was determined as compared to control groups (p<0.001) and an 86% reduction when compared to Aβ 1-40 immunized mice (p=0.03) (See Table 1, Fig 1).

In order to determine whether the effect was unique to MOG-induced EAE, EAE was induced with proteolipid protein (PLP) peptide (amino acids 139-151) in CFA. A 76% reduction of staining for Aβ fibrils was found and a 70% reduction in Aβ levels in animals with PLP induced EAE (p<0.02) (See Table 2, Fig 3A). In order to induce EAE with PLP, Tg2576 mice as described by Hsiao, K. et al., Science 274 (5284):99-102 (1996) were utilized, which are on a B6/SJL background. Similar results were found with Tg2576 mice and J20 immunized mice described by Mucke et al., Ann NY Acad Sci (777) 82-88 (1996), to induce MOG-EAE (See Table 1). These results demonstrate that the observation was not related to the antigen used for EAE induced or the animal model of AD studied, nor the genetic background of the animal, nor the gender (50% male/female). No changes were observed in animals immunized with BSA in CFA. Of note, there was no difference in the total Aβ (amyloid) load in J20 mice greater than 13 months in age when compared to Tg2576 mice greater than 16 months of age.

In previous studies of immune approaches for the treatment of the mouse model of AD in which animals were immunized with Aβ peptide formulated in CFA, anti-aggregating β amyloid antibodies have been shown to have a role both *in vitro* by Solomon, B. et al, Proc Natl Acad Sci 94:4109-12 (1997), and *in vivo* by Weiner, H. L. et al., Ann Neurol 48, 567-79 (2000), and Schenk, D. et al., Nature 400, 173-7 (1999), in reducing amyloid load, and their activity has been linked to a specific epitope in the N-terminal region of Aβ (Frenkel, D. et al., Neuroimmunol 88, 85-90 (1998), and Frenkel, D. et al., Proc Natl Acad Sci U S A 97,11455-9 (2000)). Antibody levels were measured against Aβ in animals immunized with MOG or PLP to determine if there was cross reactivity with Aβ and Aβ antibody titers as compared to those from animals immunized with Aβ. As shown in Tables 1 and 2, anti-Aβ antibodies in animals immunized with MOG or PLP were not detected. To definitively establish that antibodies were not playing a role, 16-month-old J20 mice bred to µMT B-cell deficient mice were immunized with MOG 35-55 in CFA followed by administration of Pertussis toxin. As shown in Table 1 and Figure 1, µMT B-cell deficient APP+ mice, had a 90% reduction in amyloid compared to control as measured by either ThS staining (p<0.001) or by ELISA for total brain amyloid (p<0.001). These results indicate that the reduction of Aβ following MOG immunization occurred by an antibody independent mechanism.

Previous studies have suggested that activated microglia may play an important role in clearing Aβ in vivo (Schenk, D. et al., Nature 400, 173-7 (1999), Rogers, J. et al., Glia 40, 260-9 (2002), Mitrasinovic, O.M. et al., Neurobiol Aging 24, 807-15 (2003), Webster, S.D. et al., Exp Neurol 161, 127-38 (2000), Bacskai, B.J. et al., J Neurosci 22, 7873-8 (2002), Nicoll, J.A. et al., Nat Med 9, 448-52 (2003), Akiyama, H. & McGeer, P.L., Nat Med 10, 117-8; author reply 118-9 (2004)). Activated microglia (or microglia-like cells) may be further distinguished based upon being brain derived or originating from outside the brain, i.e., peripherally, such as neutrophils and macrophages. Procedures to distinguish brain derived microglia from peripheral neutrophils and macrophages are known in the art. To investigate the potential role of microglia activation in Aβ clearance, the brains of APP tg mice immunized (by foot pad injection or intranasally) with MOG/CFA were stained with CD1 1b, a marker of activated microglia. As shown in Figures 3A and 4 and Table 4 immunostaining of the hippocampus of APP tg MOG-immunized mice revealed increased numbers of activated microglia (349 ± 34.3 cells/hippocampus region) which co-localized with amyloid plaques (Table 4). Only minimal staining was observed in control animals immunized with BSA/CFA (76 ± 17 cells/hippocampus region) (Table 4). Intermediate levels of microglia activation were observed in animals immunized with Aβ/CFA (106 ± 27 cells/hippocampus region). Furthermore, increased levels of microglia activation were also observed in µMT B-cell deficient mice (p<0.001), and animals immunized with PLP (p<0.001) (Fig. 3A, Table 4).

According to the first series of experiments described just above, administration of MOG/PLP plus CFA (followed by administration of pertussis toxin) was associated with a reduction of AB but was coincident with unwanted EAE. In these experiments pertussis toxin is used to open the blood-brain barrier for delivery of CFA formulated compounds into the brain. Therefore, in order to evaluate whether the reduction in Aβ was directly related to the induction in EAE and since MOG, PLP and GA have been studied in relation to MS related EAE, the effects of immunization of APP mice with glatiramer acetate (GA), which is a random amino acid copolymer of alanine, lysine, glutamic acid, and tyrosine that is effective in suppression of EAE and is an approved and widely used treatment for relapsing forms of MS (Teitelbaum, D. et al., J. Neural Transm Suppl 49, 85-91 (1997)) were evaluated.

Although as initially performed, administration of certain antigen/adjuvant mixtures provided herein is followed by an administration (injection) of pertussis toxin; it is fully appreciated that certain other compositions described herein may be administered without administration of pertussis toxin. Indeed, proteosome based compositions such as IVX-908 with or without GA are administered without the administration of pertussis toxin. In fact, pertussis toxin was not used at any time for the nasal delivery of any proteosome based composition described herein.

Mice were thus immunized (by foot pad injection) with 100 µg GA in CFA and immediately thereafter and at 48 hours received an i.p. injection of 150 ng of pertussis toxin. Fifty days post immunization, mice were sacrificed. Glatiramer acetate immunization led to a 92% reduction in amyloid fibril in the hippocampus region vs. untreated controls (p<0.01) and a 70% reduction of total amyloid load (p<0.01) (See Table 1, Fig 1). There was no clinical EAE in animals immunized with GA/CFA plus pertussis toxin.

As CFA cannot be administered to human subjects, the effect of nasal vaccination with glatiramer acetate in a mouse model of AD with adjuvants other than CFA was investigated; animals were treated with nasally administered glatiramer acetate alone or together with a mucosal adjuvant. A proteosome based mucosal adjuvant IVX-908 (Protollin) comprised of a non-covalent formulation of *Neisseria meningitides* outer membrane proteins (proteosomes) and LPS from *Shigella flexneri,* which has been used both in humans (Fries, L.F., et al., Infect Immun 69:4545-53 (2001)) and mice (Plante, M. et al., Vaccine 20, 218-25 (2001)), was prepared. Mice received multiple treatments of the proteosome adjuvant the first week and then were boosted on a weekly basis for the next five weeks after which neuropathologic analysis was performed. As controls, animals were nasally treated with IVX-908+BSA, IVX-908 alone or GA alone. Unexpectedly, nasal administration of GA formulated with IVX-908 resulted in an 84% reduction of thioflavin-positive fibrillar amyloid in the hippocampus (p<0.001 vs. control) and reduction of 70% compared to IVX-908 alone (p<0.01) (Table 3). In terms of total brain Aβ levels, a 73% reduction was observed following nasal administration of glatiramer acetate in IVX-908 (p<0.001 vs. control) and a 45% reduction using IVX-908 alone (p<0.002) (Table 3, Figure 2). In addition, activated microglia were detected surrounding the amyloid plaques in treated animals. Nasal administration of GA alone did not affect Aβ fibrils though there was a slight reduction of total Aβ levels in the brain (p=0.044 vs. control). Nasal administration of BSA+IVX-908 or IVX-908 alone resulted in a 50% reduction of total amyloid load (p=0.02 vs. control) although there was no effect on fibrillar Aβ staining. As opposed to injection with CFA, nasal administration of IVX-908 alone or as formulations with GA or BSA did not induce EAE in any animal in these experiments.

No microglial activation in APP non-transgenic mice was found in any of the immunization protocols: parenterally with CFA/PT plus GA, nasally with IVX-908 plus GA (See Fig 3B), or nasally with IVX-908 alone. These results suggest that the activation of microglia following administration (immunization) with GA formulated with IVX-908 or IVX-908 alone may be dependent on the presence of amyloid deposition which may serve to prime endogenous microglia for activation.

Although there is no known cross-reactivity between GA and Aβ and we did not observe anti-Aβ antibodies in either GA treated or EAE animals, it is possible that immunization with GA+IVX-908 could have resulted in priming of Aβ reactive T cells. We measured T cell proliferative responses and cytokine production (IL-2, IFN-γ, IL-6) after 7 weeks of weekly treatment with GA+IVX-908 (at which time the experiment was terminated) by stimulating splenic T cells with Aβ(1-40). We found no priming of Aβ reactive T cells as measured by proliferation: Counts per minute to Aβ for untreated = 3315 ± 1682 cpm; for GA+IVX-908 = 4516 ± 1412 cpm (background counts were 100-300). Stimulation index (GA+IVX-908/untreated) =1.37; a minimal simulation index of greater than 2.5 is considered positive. Furthermore, we did not find secretion of IL-2, IFN-γ, IL-6 above background in these cultures. This lack of T cell response to Aβ is consistent with our not detecting anti-Aβ antibodies as T cell help is required for production of antibodies. Similarly, we did not find priming to Aβ in EAE animals.

To examine the effect of GA+IVX-908 treatment on other brain sites besides the hippocampus, we investigated the olfactory bulb and the cerebellum. We stained the olfactory bulb for Aβ, CD11b and fibrinogen and obtained similar results as those observed in the hippocampus (Figure 8). Following nasal administration of GA+IVX-908 we found an increased number of activated microglia as compared to control. The activation also occurred in animals with EAE, but was associated with leakage in the blood brain barrier (BBB) as measured by staining for fibrinogen.

When we examined the cerebellum, we did not find increased activation of microglial staining in GA+IVX-908 treated animals suggesting that the increased activation is restricted to areas with Aβ deposition. Furthermore, no activation of microglia was observed anywhere in the brains of non-transgenic littermates following GA+IVX-908 which further demonstrates that the increased activation is restricted to areas with Aβ deposition (see Figure 3b).

To better understand the mechanism of the clearance we observed, we stained for CD68 expression, which is highly expressed on activated macrophages from the periphery as opposed to brain microglia. As shown in Figure 9, we obtained higher staining for CD68 in animals with EAE as compared to those treated with GA+IVX-908. This pattern of staining shows the migration of macrophages from the choroids plexus to the surrounding brain parenchyma including the cerebellum and cortex. In GA+IVX-908 treatment, there is increased expression of CD68 primarily in the choroids plexus space. This suggests that the CD1 1b+ cells responsible for clearance of Aβ in animals with EAE migrate to the CNS from the periphery and are associated with neuronal toxicity whereas the CD1 1b+ cells in GA+IVX-908 treated animals are primarily endogenous microglial cells and are associated with clearance of Aβ without evidence of direct toxicity. As further support for this interpretation, we found that there is increased expression of CD68+ cells in the cerebellum of animals with EAE but not in GA+IVX-908 or untreated animals (not shown). Moreover, activated CD11b+ cells following GA+IVX-908 treatment were only found in regions where there was accumulation of amyloid.

As shown in Table 4 and Figure 4, the reduction of Aβ fibrils in the hippocampus was strongly correlated with increased numbers of both activated microglial cells, as shown by CD11b staining, (r=-0.7 CD11b vs. Aβ fibrils), and IFN-γvs. Aβ fibrils). There was a strong correlation between CD11b cells and IFN-γ cells we observed increased numbers of microglia immunoreactive for macrophage colony-stimulating factor receptor (M-CSFR) in treated animals as compared to control (p<0.02) (Table 4). We observed a reduction in TGF-β and the percentage of Aβ fibril in the hippocampus region (r=0.91). No significant changes were observed in IL-10 immunoreactive cells between control and GA+IVX-908 treated animals though animals with EAE had less IL-10 than controls.

The following experiments were performed in order to evaluate whether treatment with GA plus IVX-908 induces neuronal cell toxicity or other potential negative effects: i) determine the level of GFAP, a measure of astrocytosis (toxicity) consistent with neuronal cell damage; ii) measure the expression level of SMI32, a marker for the phosphorylation of neurofilaments and known to increase with neuronal cell damage; iii) conduct a TUNEL assay as a means of measuring apoptotic cell death; iv) and determine the level of iNOS, an enzyme shown to be up-regulated under conditions of neuronal cell stress.

Results from the GFAP assay show that astrocytosis occurred in control untreated animals (area of activated astrocytes in hippocampus as measured by GFAP+ cells) (Figure 5). Astrocytosis was reduced in GA+IVX-908 given nasally (3.1%; p=0.039 vs. control). In contrast, there was no reduction in astrocytosis in EAE animals. These results indicate that clearance of Aβ as a consequence of treatment with GA+IVX-908 is less toxic (associated with less astrocytosis) than that observed in animals with EAE even though EAE is also associated with a reduction in Aβ deposits.

Results from experiments measuring SMI32 (Figure 6) show that SMI32 positive cells having an abnormal ovoid morphology are associated with neuritic plaques in untreated control animals. Animals with EAE show an increase in the number of SMI32 positive cells with an abnormal ovoid morphology throughout the brain (in association with inflammation) including the cerebellum (although no neuritic plaques were observed). In contrast, animals treated with GA+IVX-908 show a reduction in the number of SMI32 positive cells (with an abnormal ovoid morphology) associated with neuritic plaques. These experiments suggest that GA+IVX-908 treatment is not associated with toxicity as measured by SMI32.

Results from experiments measuring apoptotic cell death using a standard TUNEL assay (Figure 6) show no TUNEL staining in control animals and increased TUNEL staining in the cortex of EAE animals. No TUNEL staining was observed in GA+IVX-908 treated animals. In addition, assays measuring iNOS indicate that iNOS is up-regulated in mice with EAE but not in animals treated with GA+IVX-908. No damage to vascular structures was detected in either GA-IVX-908 treated or EAE animals.

Collectively, these data indicate that even though there was clearance of Aβ in animals with EAE as well as in animals treated with GA +IVX-908, the latter was not associated with neuronal cell toxicity (Table 4 and Figure 6).

However, although activation of microglia following immunization with IVX-908 alone appears to require the presence of Aβ deposits, such immunization did not result in removal of such Aβ deposits (by activated microglia), but rather there was a preferential reduction in the amount of total amyloid burden. Such results may suggest the possibility that there are two populations of microglia which may be activated, or, as an alternative possibility, that microglia may be activated to different degrees, partially or fully, where fully activated microglia are capable of removing pre-existing Aβ plaques, and partially activated microglia participate in sequestration of soluble Aβ peptide. In consideration of the notion that soluble Aβ aggregates into insoluble Aβ plaques, such treatment with IVX-908 alone may slow or prevent continued formation of Aβ plaques, having benefit to a disease bearing subj ect.

Table 4 and Figure 3B, demonstrate that the reduction of Aβ fibrils in the hippocampus was correlated with an increased number both of activated microglial cells (as shown by CD11b+ immunohistochemical staining) and of IFN-γ secreting cells. In addition, there were increased numbers of macrophage colony-stimulating factor receptor (M-CSFR) positive microglia. It has been reported that increased expression of M-CSFR on mouse and human microglia accelerate phagocytosis of aggregated amyloid both through macrophage scavenger receptors and by increasing microglial expression of FcR gamma receptors (Mitrasinovic, O.M. & Murphy, G.M., Jr., Neurobiol Aging 24, 807-15 (2003)). However, clearance of amyloid in the µMT B-cell deficient mice also had increased staining for M-CSFR, therefore the observed effects are via a non-Fc-mediated mechanism (Bacskai, B.J. et al., J Neurosci 22, 7873-8 (2002)). In association with increases in IFN-γ, a reduction in TGF-β expression was observed, suggesting that TGF-β somehow modulates the ability of soluble Aβ to aggregate into plaques, and it is further believed that TGF-β is associated with increased amyloid deposition (Wyss-Coray, T. et al., Nature 389, 603-6 (1997)). The decrease in TGF-β also may facilitate amyloid clearance. No significant changes were observed in IL-10 expression. Microglial activation might be associated with increased numbers of T cells, which may have played a role in promoting microglial activation as there was a correlation between numbers ofT cells and numbers of IFN-γ secreting cells.

In order to confirm that the CD1 1b+ cells detected in these experiments were activated macrophages or microglia and not neutrophils, samples were incubated with a monoclonal antibody recognizing F4/80, a cell surface structure that can be detected on the surface of activated macrophages and microglia but not on neutrophils (polymorphonuclear leukocytes). The expression of F4/80 increases with maturation of macrophages and microglia. The results of these experiments indicate that all CD11b+ cells detected in these experiments also stained positive for F4/80 (not shown), thereby indicating that these CD1 1b+ cells were activated macrophages or microglia, not neutrophils. In addition, the CD1 1b+ cells that co-localized with Aβ plaques by H&E staining (Figure 4) have a mononuclear morphology and not the polymorphonuclear morphology characteristic of neutrophils.

In yet another series of experiments it was shown that microglia cell activation following nasal administration of GA + IVX-908 correlated with an increase in the number of T cells, as determined by CD3 staining. These results suggest a possible role for T cells in promoting microglia cell activation, as there was a correlation between the number of T cells and the number of IFN-γ secreting cells detected r= 0.88 (Table 4). Additionally, it has been reported that TGF-β may either increase or reduce Aβ fibril formation in APP tg-mouse Wyss-Coray, T. et al Nature 389, 603-6 (1997); Wyss-Coray et al. Nat. Med. 7:612-8 (2001). In the experiments reported here, a reduction in TGF- β expression was observed in MOG (p<0.001) and GA+IVX-908 (p<0.001) treated mice compared to control (Table 4); there was a strong correlation in the reduction TGF- β and the percentage of Aβ fibrils in the hippocampus region (r=0.95). No significant changes were observed in IL-10-immunoreactive cells (not shown).

A decrease in the level of total brain Aβ was found when IVX-908 alone was given nasally (Table 3) (p=0.02 vs. untreated), but unlike IVX-908 formulated with GA, there was no effect of IVX-908 alone on clearance of thioflavin positive Aβ fibrils. IVX-908 is a proteosome based adjuvant composed of *N. meningitides* outer membrane proteins (OMPs) and exogenously added lipopolysaccharide (LPS). *Neisseria meningitidis* OMP 2 (porin B) and LPS/LOS are known to interact with TLRs displayed on the surface of certain cell types concerning the innate and/or adoptive immune system. It is possible that such interactions are required, at least in part, for the observed activity of IVX-908 and/or GA formulated with IVX-908, as set forth herein. The effect observed with IVX-908 may be related to reports that direct injection of LPS into the hippocampus can cause a reduction of non-fibril Aβ load but not fibril Aβ deposits (DiCarlo, G. et al., Neurobiol Aging 22, 1007-12 (2001)). However, it must be noted that the route of administration in these experiments, direct injection into the brain, is dramatically distinct from the nasal route of administration for delivery of proteosome based formulations described herein. Contrastingly, other reports indicate that although LPS (inflammation) given intraperitonealy may stimulate the activation of microglial cells, an increase in total amyloid was also observed, LPS-induced neuroinflammation increases the intracellular accumulation of amyloid precursor protein and Aβ peptide (Sheng et al., Neurobiology of Disease 14:133-145 (2003), and references cited therein). Furthermore, it is appreciated that direct injection of LPS is expected to be toxic.

In experiments discussed above, CD11b+ cells were shown to be activated microglia or macrophages but not neutrophils by the absence of F4/80 signal. However, in order to determine if these CD11b+ cells could be further distinguished as an activated microglia as opposed to an activated macrophage, samples were evaluated for the presence of CD68, a cell marker that is highly expressed on activated macrophages from the periphery but is not highly expressed on the surface of microglia cells originating from the brain. As shown in figures 8 and 9, we obtained higher staining for CD68 (macrophage are CD11b+ and CD68+) in EAE animals compared to samples derived from animals treated with GA+IVX-908; indicating that these CD 11b+ CD68+ cells are macrophages which have migrated into the brain parenchyma, including the cerebellum and cortex, from the periphery (subarachnoid space). In contrast, following GA-IVX-908 treatment, there is an increase in CD68 expressing macrophage, but these cells remain primarily localized to the subarachnoid space. The results from these experiments suggest that the CD1 1b+ cells implicated in the clearance of Aβ in EAE animals migrate into the CNS from the periphery and are associated with neuronal toxicity; whereas, the CD1 1b+ cells detected following GA-IVX-908 treatment are primarily endogenous microglia cells and are associated with clearance of Aβ without evidence of direct toxicity. To further support this interpretation, we found that there is increased expression of CD68+ cells in the cerebellum of EAE animals but not in GA-IVX-908 treated or untreated animals (data not shown). Moreover, the activated CD11b+ cells following GA-IVX-908 treatment were only found in regions where there was an accumulation of amyloid.

We also found increased levels of Aβ in the serum of animals administered GA+IVX-908 as compared to untreated animals, suggesting that GA+IVX-908 leads to clearance of Aβ from the brain regions and that this Aβ may then be found in the periphery. However, such a redistribution of Aβ was not observed in animals having EAE, which, as described above, may relate to the source of activated CD11b+ cells. In addition, the number of CD1 1b+ CD68+ activated macrophage lining brain capillaries and the choroid plexus was increased in animals administered GA-IVX-908 compared to untreated animals (Figure 9). These findings are consistent with the elevated levels of Aβ detected in serum samples obtained from GA-IVX-908 treated animals, and a concomitant decrease in amyloid angiopathy in GA-IVX-908 treated animals in association with CD11b+ cells (Figure 8).

Unexpectedly, it appears the final common pathway of amyloid (*e.g*., Aβ plaque) clearance may be via activated microglia. In EAE, IFN-γ Th1 type myelin reactive T cells are apparently activated in the periphery by immunization with MOG or PLP plus CFA and these T cells migrate to the brain where they release IFN-γ (a Th1 cytokine) and activate microglia. As a consequence, encephalomyelitis and paralysis of animals is caused by the damage to myelin and underlying axons. Immunization with BSA/CFA in the periphery does not lead to Aβ clearance, as BSA specific Th1 type cells do not accumulate in the brain. Peripheral immunization with glatiramer acetate in CFA induces GA specific T cells that accumulate in the brain due to the cross reactivity of GA with MBP. The cells are able to secrete IFN-γ and thus activate microglia, but are unable to cause EAE because of altered affinity for MBP and the concomitant secretion of anti-inflammatory cytokines.

We have demonstrated clearance of Aβ in association with microglia activation. It should be pointed out that microglial activation can have both positive and negative effects (Monsonego, A., and Weiner, H.L., Immunotherapeutic approaches to Alzheimer's disease, Science 302:834-838 (2003)). Microglia represent a natural mechanism by which protein aggregates and debris can be removed from the brain and there are reports that microglial activation following Aβ immunization or stroke may lead to Aβ clearance (see Nicoll, J.A., et al., Neuropathology of human Alzheimer disease after immunization with amyloid-beta peptide: a case report, Nat Med., 9:448-452 (2003); Akiyama, H., and McGeer, P.L., Specificity of mechanisms for plaque removal after A beta immunotherapy for Alzheimer Disease, Nat Med.,10:117-118; author reply 118-119 (2004); and Wyss-Coray, T., et al., Prominent neurodegeneration and increased plaque formation in complement-inhibited Alzheimer's mice., Proc Natl Acad Sci., 99:10837-10842 (2002)). In animal studies, Wyss-Coray and colleagues demonstrated that there is prominent neurodegeneration and increased plaque formation in the complement-inhibited AD mouse model, in which microglia were significantly less activated than in wild type AD mice at the same age. This supports the concept that activated microglia have a beneficial role in decreasing amyloid load without major neurotoxicity in the AD mouse model.

Nasal administration of IVX-908 alone leads to reduction of amyloid as it is able to activate microglia, though not as efficiently as IVX-908 compositions formulated with GA which, in addition, may activate T cells. No microglia activation was observed with GA given peripherally with CFA or intranasally with NX-908 in non-transgenic animals. It has been reported that Aβ deposition leads to slight activation of microglia surrounding Aβ plaque and it appears that this activation is required in order for microglia to be further activated by IVX-908 plus GA. It is possible that slight activation of microglia is associated with the expression of IFN-γ or toll-like receptors which prime microglia for further activation (Sasaki, A. et al., Virchows Arch., 441(4):358-67 (2002)).

These findings have relevance to potential mechanisms for plaque removal observed in humans following immunization with Aβ1-42 in a Th1 type adjuvant (QS21), Nicoll, J.A. et al., Nat Med 9, 448-52 (2003) reported autopsy findings from an AD patient vaccinated with Aβ1-42 given parenterally with adjuvant QS21 which resulted in widespread meningoencephalitis, infiltration of the brain by macrophages, and a reduction of amyloid deposits in the neocortex. Akiyama and McGeer reported a similar reduction of senile plaques in a cortical area affected by incomplete ischemia in a case of AD and suggest that their findings and those reported by Nicoll, et al may be related to phagocytosis of amyloid by highly reactive microglia in an antibody dependent manner. Furthermore, by using TUNEL staining (a marker for apoptotic cells) or NeUN immunostaining (a marker for viability of neurons) no evidence of toxicity was observed in GA plus IVX-908 or IVX-908 alone immunization compared to untreated mice.

A novel immune therapeutic approach for the treatment of Alzheimer's disease is provided herein that is antibody independent and is mediated by activated microglia. By combining a drug used to treat multiple sclerosis with a nasal adjuvant (IVX-908); microglia appear to be activated to clear Aβ-fibril plaques and reduce total amyloid burden using two compositions (GA and proteosome based adjuvants) that previously have been used in humans for other indications with no toxicity. Given that studies in animals have demonstrated that reduction of Aβ plaque is associated with cognitive improvement, nasal vaccination with IVX-908 formulated with GA is an effective therapy for patients with Alzheimer's disease.

**Table 1. Effect of subcutaneous immunization on total and fibrillar Aβ in the brains of J20 APP transgenic mice.**

| | Mice per group | EAE score | Anti-Aβ Antibody* | Total brain Aβ (ng/ml) | % Area of thioflavin positive Aβ plaques (hippocampus) |
|---|---|---|---|---|---|
| Control** | 8 | 0 | 0 | 125.6±15.9 | 2.6±0.4 |
| Aβ | 8 | 0 | 0.75 | 60.8±16 **^{a}** | 0.83±0.3 **^{a}** |
| MOG | 10 | 2.5±0.8 | 0 | 8.4±2.1 **^{b}** | 0.22±0.1 **^{b}** |
| MOG B-deficient | 6 | 2.7±0.4 | 0 | 11.4±2.7 **^{b}** | 0.27±0.04 **^{b}** |
| GA | 5 | 0 | 0 | 39.1±12.8 **^{c}** | 0.26±0.1 **^{d}** |

| | | | | | |
|---|---|---|---|---|---|
| * Results are presented as level of OD at titer of 1:500 IgG. ** Control combines untreated (n=5) and BSA/CFA treated (n=3) animals as there was no difference between these groups. For total brain Aβ: untreated=126.7 ± 19.5; BSA/CFA 123.7± 33.2. For % area of thioflavin positive Aβ plaques: untreated: 2.8± 0.5; BSA/CFA =2.2±0.9. ^{a}p<0.05 vs. control. ^{b}p<0.001 vs. control; p<0.05 vs. Aβ. ^{c}p<0.01 vs. control. ^{d}p<0.01 vs. control; p=0.05 vs. Aβ. | | | | | |

**Table 2. Effect of PLP immunization on total and fibrillar Aβ in the brains of Tg2576 APP transgenic mice.**

| | Mice per group | EAE score | Anti-Aβ Antibody* | Total brain Aβ (ng/ml) | % Area of thioflavin positive Aβ plaques (hippocampus) |
|---|---|---|---|---|---|
| Control | 5 | 0 | 0 | 133.1±32.7 | 2.67±0.4 |
| PLP | 5 | 1.7±0.7 | 0 | 47.7±18.4 **^{a}** | 0,68±0,2**^{b}** |

| | | | | | |
|---|---|---|---|---|---|
| *The results are presented as level of OD 450 at titer of 1:500 IgG. ^{a}p<0.02 vs. control (untreated mice). ^{b}p<0.002 vs. control. | | | | | |

**Table 3. Effect of nasal immunization on a total and fibrillar Aβ in the brains of J20 APP transgenic mice.**

| | Mice per group | EAE score | Anti-Aβ Antibody* | Total brain Aβ (ng/ml) | % Area of thioflavin S positive Aβ plaques (hippocampus) |
|---|---|---|---|---|---|
| Control* | 8 | 0 | 0 | 125.6 ± 15.9 | 2.6 ± 0.4 |
| GA | 4 | 0 | 0 | 97.6 ± 15.5 | 2.06 ± 0.8 |
| IVX-908 | 7 | 0 | 0 | 63.5 ± 7,7**^{a}** | 1.5 ± 0.2 |
| GA+IVX-908 | 8 | 0 | 0 | 38.7 ± 6.8**^{b}** | 0.48 ± 0.09 ^{c} |

| | | | | | |
|---|---|---|---|---|---|
| **Footnote** * Results are presented as level of OD 450 at titer of 1:50 IgG. ^{a}p<0.02 vs. control, p<0.02 vs. GA. ^{b}p<0.001 vs. control and GA, p<0.04 vs. IVX-908. ^{c}p<0.001 vs. control, p<0.002 vs. GA, p<0.002 vs. IVX-908. | | | | | |

**Table 4. Immunohistochemistry of hippocampus in immunized animals***

| | Number of cells per hippocampal region* | | | | |
|---|---|---|---|---|---|
| | CD11b **^{a}** | CD3**^{b}** | M-CSFR **^{c}** | IFN-γ**^{d}** | TGF-β **^{e}** |
| | | | | | |
| **Control** | 76 ± 17 | 5 ± 3 | 11 ± 4 | 7 ± 3 | 73 ± 12 |

| **Subcutaneous** **(CFA/P.T.)** | | | | | |
|---|---|---|---|---|---|
| **Aβ** | 106 ± 27 | 10 ± 2 | 34 ± 11 | 9 ± 2 | 41 ± 5 |
| **MOG** | 349 ± 34.3 ^{iv} | 142 ± 19^{iv} | 79 ± 5ⁱ | 109 ± 7 ^{iv} | 20 ± 5ⁱⁱⁱ |
| **MOGB-deficient** | 462 ± 23^{iv} | 195 ± 12^{iv} | 77 ± 6ⁱ | 119 ± 18^{iv} | 22 ± 8ⁱⁱ |
| **GA** | 227 ± 61ⁱ | 61 ± 12ⁱ | 57 ± 8 | 85 ± 11^{iv} | 31 ± 7 |

| **NASAL** | | | | | |
|---|---|---|---|---|---|
| **GA** | 136 ± 12 | 35 ± 19 | 30 ± 9 | 53 ± 3ⁱ | 77 ± 10 |
| **IVX-908** | 406 ± 16^{iv} | 55 ± 9ⁱ | 81 ± 16ⁱ | 92 ± 4^{iv} | 40 ± 11 |
| **GA+** | 451 ± 48^{iv} | 67 ± 9ⁱⁱ | 119 ± 30^{iv} | 81 ± 10ⁱⁱⁱ | 14 ± 2^{iv} |
| **IVX-908^{g}** | | | | | |

| | | | | | |
|---|---|---|---|---|---|
| **Table 4 footnote:** * Data represents quantification of three sections for each treatment and six sections for the control (3 untreated + 3 BSA/CFA treated as table 1). **^{a}** r = - 0.7 CD11b vs. % Area of Aβ fibril. **^{b}** r = - 0.65 CD3 vs. % Area of Aβ fibril; r= 0.74 CD3 vs. CD11b. **^{c}** r = - 0.7 M-CSFR vs. % Area of Aβ fibril; r= 0.92 M-CSFR vs. CD11b. **^{d}** r = - 0.8 IFN- γ vs. % Area of Aβ fibril; r= 0.9 IFN- γ vs. CD11b; r= 0.85 IFN- γ vs. CD3. ^{e} r = 0.91 TGF-β vs. % Area of Aβ fibril; r=- 0.77 TGF-β vs. CD11b; r=- 0.6 TGF-β vs. CD3. ^{f} r=0.67 IL-10 vs. % Area of Aβ fibril; r=0.4 IL-10 vs. CD11b. ^{g} p=0.0007 CD11b vs. GA; p<0.05 IFN-γvs. GA; p=0.0011 TGF-β vs. GA. ⁱ p<0.05 vs. control. ⁱⁱp<0.02 vs. control. ⁱⁱⁱp<0.001 vs. control. ^{iv}p<0.001 vs. control. | | | | | |

### MATERIALS AND METHODS

**Mice.** (B6XD2)F1 (average age 14 months) or (B6XSJL)F1 APP+ (WT or µMT) (average 16 months) APP transgenic micer were housed and used in a pathogen-free facility at the Brigham and Woman's Hospital in accordance with all applicable guidelines.

**Materials.** IVX-908 (Protollin) is a non-covalent formulation of *Neisseria meningitides* outer membrane proteins (proteosomes) and LPS from *Shigella flexneri* that has been safely tested in humans, and was obtained from ID Biomedical, Montreal, Canada. Glatiramer acetate (Copaxone^{®}) is a random amino acid copolymer of alanine, lysine, glutamic acid and tyrosine that is an approved and widely used treatment for relapsing forms of MS, and was obtained from the Brigham and Women's Hospital pharmacy. MOG (35-55) and PLP (139-151) were synthesized at the Center for Neurologic Diseases, Brigham and Women's Hospital.

**Induction and clinical evaluation of EAE in APP+ mice.** (B6D2)F1 or (B6XSJL)F1 APP+ (WT or B-cell deficient µMT) and non-tg littermates were immunized in the hind footpads with 100 µg MOG(35-55), PLP 139-151 or 100 µg β-amyloid peptide (1-40) in CFA. Immediately thereafter and again 48 hours later mice received an i.p. injection containing 150 ng of pertussis toxin in 0.2 ml PBS. Animals were monitored for symptoms of EAE beginning 7 days after immunization and scored as follows: 0, no disease; 1, tail paralysis; 2, hind limb weakness; 3, hind limb paralysis; 4, hind limb plus forelimb paralysis; and 5, moribund.

**Nasal vaccination.** Glatiramer acetate: 25 µg was given on days 1, 2, 4 and 5 the first week followed by a weekly boost for six weeks. IVX-908: 1 µg/mouse was given on days 1 and 5 the first week followed by a weekly boost for six weeks. BSA+IVX-908: 25 µg of BSA plus 1 ug IVX-908 were given on days 1 and 5 the first week, and 25 µg of BSA alone was given on days 2 and 4, followed by six weekly boosts of the combination of BSA+IVX-908. GA+IVX-908: 25 µg of GA plus 1 µg IVX-908 were given on days 1 and 5 the first week, and 25 µg of GA alone was given on days 2 and 4, followed by six weekly boosts of the combination of GA+IVX-908.

**Amyloid quantification.** To quantify amyloid burden, the right hemisphere was extracted in 5.0 M guanidinium-chloride (pH 8) for 3 hours at room temperature. Dilutions were used to measure levels of insoluble (amyloid-associated) Aβ40 and Aβ42 by sandwich enzyme-linked immunosorbent assays (ELISA). To measure Aβ fibrils, the left hemisphere was fixed in 4% Brain O/N followed by 4.5% sucrose for 4 hours then 20% Sucrose for O/N at 4°C. Brains were frozen in the presence of OCT paraformaldehyde, cut to 14-µm longitudinal sections used for immunohistological staining and amyloid fibril quantification. Well-defined hippocampal regions (Bregma -1.34), were selected for quantification of the amount of amyloid fibril in plaques using thioflavin-S staining. Images (magnification x20) from these sections were collected from a 3CCD color video camera and analyzed with appropriate software (NIH; Imaging Research). The amount of amyloid fibril was expressed as a percentage per mm² hippocampal region as measured by the software.

**Immunohistology.** The staining was performed utilizing the following markers: T-cells (CD3; BD Biosciences:553057), microglia/macrophages (CD11b; Serotec:MCA74G), (C-MFR; Cymbus Biotech:21080096), IFN-γ (Pharmingen: 559065), IL-10 (Pharmingen:559063) and TGF-β (RD:AB-20-PB). Anti-amyloid antibodies (R1282) were a gift of Dennis Seloke. Brain sections were further subject to Haematoxylin staining. Sections were evaluated in a blinded manner, and controls included use of isotype-matched mAbs as previously described. For each treatment the quantification was done from the hippocampal region of three different brain sections (the same region, Bregma -1.34, that were used for ThS staining). The results are expressed as the mean of the labeled cells for each marker.

**Neuropathology.** To examine for neurotoxicity the left hemisphere was fixed in 4% paraformaldehyde overnight followed by 4.5% sucrose for 4 hours then 20% sucrose for overnight at 4°C. Brains were frozed in the presence of OCT paraformaldehyde, cut to 14-µm longitudinal sections and used for immunohistological staining. We stained for four markers used for neuronal stress and blood brain barrier integrity: GFAP (Sigma;G9269), SMI32 (Serotec), TUNEL (Roche 1 684 817), iNOS (CHEMICON:AB5382), and Fibrinogen (Dako: A0080). Astrocytosis is expressed as a percentage per mm² of the hippocampal region covered by astrocytes. Staining for iNOS, SMI32 and Fibrinogen was done as previously described (29). Staining for Terminal deoxynucleotidyl transferase-mediated dUTP nick-end labeling (TUNEL) was carried out according to manufacturer's (Roche 1 684 817) recommendations. H&E staining was carried out to identify the morphology of cells counted. The staining was performed on two consecutive sections per animal and four animals per group in a blinded fashion using Imaging Research software from the NIH in an unbiased stereological approach. Staining per group from the primary motor cortex (Bregma lateral 1.44 mm) is show in Figure 6.

**Data analysis.** All continuous and ordinal data are expressed as mean ± sem. Data comparisons were carried out using Student's t-test when two groups were compared, or one-ANOVA analysis when three or more groups were analyzed.. Values of p less than 0.05 were considered statistically significant; r values were calculated using an Excel statistical program.

All references cited herein, including patents, patent applications, and publications, are hereby incorporated by reference in their entireties, whether previously specifically incorporated or not.

Having now fully described this invention, it will be appreciated by those skilled in the art that the same can be performed within a wide range of equivalent parameters, concentrations, and conditions without departing from the spirit and scope of the invention and without undue experimentation.

While this invention has been described in connection with specific embodiments thereof, it will be understood that it is capable of further modifications. This application is intended to cover any variations, uses, or adaptations of the invention following, in general, the principles of the invention and including such departures from the present disclosure as come within known or customary practice within the art to which the invention pertains and as may be applied to the essential features hereinbefore set forth.

Embodiments of the invention are:
1. A method of treating a neurological disease or disorder in a mammal, which method comprises administering to said mammal in need of such treatment a therapeutically effective amount of a proteosome based composition.
2. The method of item 1 further comprising administering a therapeutically effective amount of glatiramer acetate either in the same or in a separate formulation with said proteosome based composition.
3. The method of item 1, wherein said neurological disease or disorder comprises deleterious protein aggregation.
4. The method item 1, wherein said neurological disease or disorder is Multiple Sclerosis.
5. The method of item 3 wherein said neurological disease or disorder is selected from the group consisting of early onset Alzheimer's disease, late onset Alzheimer's disease, presymptomatic Alzheimer's disease, Serum Amyloid A (SAA) amyloidosis, prion disease, hereditary Icelandic syndrome, senility and multiple myeloma.
6. The method of item 3, wherein treating said neurological disease or disorder results in a reduction in soluble or insoluble amyloid beta peptide, and wherein said insoluble amyloid beta peptide comprises fibrillar amyloid beta peptide.
7. The method of item 6 wherein said amyloid beta peptide is insoluble.
8. The method of item 1 wherein said neurological disease or disorder is an amyloidal disease.
9. The method of item 8 wherein said amyloidal disease is Alzheimer's disease.
10. The method of item 9 wherein said treating the amyloidal disease comprises preventing an increased amyloid load, maintaining the current amyloid load, or decreasing the amyloid load in the brain.
11. The method of item 10 wherein said amyloid is a β-amyloid.
12. The method of item 10 wherein said amyloid load includes total amyloid load and fibrillar load.
13. The method of item 1, wherein the proteosome based composition is selected from the group consisting of a proteosome based adjuvant containing an endogenous lipopolysaccharide and a proteosome based adjuvant containing an exogenous lipopolysaccharide.
14. The method of item 13, wherein said proteosome and said lipopolysaccharide are obtained from the same bacterial genus.
15. The method of item 13, wherein said proteosome and said lipopolysaccharide are obtained from different bacterial genuses.
16. The method of item 13, wherein said proteosome is from *Neisseria meningitides,* and said lipopolysaccharide is from *Shigella flexneri.*
17. The method of item 1 further comprising a pharmaceutically acceptable diluent, excipient, stabilizer or carrier.
18. A method for treating a neurological disease or disorder in a mammal, which method comprises administering to said mammal in need of such treatment a therapeutically effective amount of Glatiramer Acetate in a sub-micron emulsion or nanoemulsion.
19. The method item 18 wherein said neurological disease or disorder is a cell- mediated autoimmune disease or disorder.
20. The method of item 19, wherein said cell-mediated autoimmune disease or disorder is Multiple Sclerosis.
21. A composition comprising Glatiramer Acetate in a sub-micron or nanoemulsion.
22. A composition comprising Glatiramer Acetate and a proteosome based composition.
23. A method for treating a neurological disease or disorder in a mammal in need of such treatment comprising administering a therapeutically effective amount of a composition which elicits an antibody independent response in said mammal;
wherein said composition comprises any of the following;
(a) a proteosome based composition;
(b) a proteosome based composition in conjunction with or formulated with a Glatiramer Acetate composition;
(c) a Glatiramer Acetate composition formulated with a sub-micron emulsion; or
(d) a Glatiramer Acetate composition formulated with a nanoemulsion.

## Claims

**1.** A proteosome based composition for use in the treatment of a neurological disease or disorder in a mammal.

**2.** Use of a proteosome based composition for the manufacture of a medicament for treating a neurological disease or disorder in a mammal.

**3.** The proteosome based composition of claim 1 or the use of claim 2, further comprising glatiramer acetate, wherein glatiramer acetate is either in the same formulation with said proteosome based composition or in a separate formulation.

**4.** The proteosome based composition of claim 1 or 3, or the use of claim 2 or 3, wherein said neurological disease or disorder comprises deleterious protein aggregation.

**5.** The proteosome based composition of claim 1 or 3, or the use of claim 2 or 3, wherein said neurological disease or disorder is Multiple Sclerosis or an amyloidal disease, wherein said amyloidal disease preferably is Alzheimer's disease.

**6.** The proteosome based composition of claim 4, or the use of claim 4, wherein said neurological disease or disorder is selected from the group consisting of early onset Alzheimer's disease, late onset Alzheimer's disease, presymptomatic Alzheimer's disease, Serum Amyloid A (SAA) amyloidosis, prion disease, hereditary Icelandic syndrome, senility and multiple myeloma.

**7.** The proteosome based composition of claim 5, or the use of claim 5, wherein treatment of the amyloidal disease comprises preventing an increased amyloid load, maintaining the current amyloid load, or decreasing the amyloid load in the brain, wherein said amyloid preferably is a β-amyloid.

**8.** The proteosome based composition of claim 1 or 3, or the use of claim 2 or 3, wherein the proteosome based composition is selected from a proteosome based adjuvant comprising an endogenous lipopolysaccharide and a proteosome based adjuvant comprising an exogenous lipopolysaccharide.

**9.** The proteosome based composition of claim 8, or the use of claim 8, wherein said proteosome and said exogenous lipopolysaccharide are obtained from
(a) the same bacterial genus; or
(b) different bacterial genera.

**10.** The proteosome based composition of claim 8, or the use of claim 8, wherein said proteosome is from *Neisseria meningitidis,* and said lipopolysaccharide is from *Shigella flexneri.*

**11.** Glatiramer Acetate in a sub-micron emulsion or nanoemulsion for use in treating a neurological disease or disorder in a mammal.

**12.** Use of glatiramer acetate in a sub-micron emulsion or nanoemulsion for the manufacture of a medicament for treating a neurological disease or disorder in a mammal.

**13.** The glatiramer acetate of claim 11, or the use of claim 12, wherein said neurological disease or disorder is a cell-mediated autoimmune disease or disorder, wherein said cell-mediated autoimmune disease or disorder preferably is multiple sclerosis.

**14.** A composition comprising
(a) glatiramer acetate in a sub-micron or nanoemulsion; or
(b) glatiramer acetate and a proteosome based composition.

**15.** A method for treating a neurological disease or disorder in a mammal in need of such treatment comprising administering a therapeutically effective amount of a composition which elicits an antibody independent response in said mammal;
wherein said composition comprises any of the following;
(a) proteosome based composition;
(b) a proteosome based composition in conjunction with or formulated with a Glatiramer Acetate composition;
(c) a Glatiramer Acetate composition formulated with a sub-micron emulsion; or
(d) a Glatiramer Acetate composition formulated with a nanoemulsion.
